# EUROPEAN PATENT SPECIFICATION

(11) **EP 2 502 543 B1**
(45) Date of publication and mention of the grant of the patent: **27.09.2017**
(21) Application number: 10831376.8
(22) Date of filing: 20.08.2010
(51) Int. Cl.: A61B 1/00, A61B 1/04, A61B 5/07

(54) **CAPSULE MEDICAL DEVICE GUIDANCE SYSTEM**
SYSTEM ZUM FÜHREN EINER VERKAPSELTEN MEDIZINISCHEN VORRICHTUNG
SYSTÈME DE GUIDAGE DE DISPOSITIF MÉDICAL À CAPSULE

(30) Priority: 19.11.2009 JP 2009264040
(43) Date of publication of application: 26.09.2012
(73) Proprietor: Olympus Corporation, Tokyo 192-8507 (JP); Siemens Healthcare GmbH, 91052 Erlangen (DE)
(72) Inventor: KAWANO, Hironao, Hachioji-shi, Tokyo 192-8507 (JP); KELLER, Henrik, 91054 Erlangen (DE)
(74) Representative: Schicker, Silvia
(86) International application number: PCT/JP2010/064108
(87) International publication number: WO 2011/061977

(56) References cited:
- WO-A1-2007/077922
- WO-A1-2008/082005
- DE-B3-102008 018 723
- JP-A- 2006 087 521
- JP-A- 2006 263 167
- JP-A- 2006 509 574
- JP-A- 2007 151 729
- JP-A- 2007 195 961
- JP-A- 2009 000 376
- JP-A- 2009 247 494
- US-A1- 2007 221 233
- US-A1- 2009 253 954

## Description

### TECHNICAL FIELD

The present invention relates to a capsule medical device guidance system that guides a capsule medical device inserted in a subject.

### 2. Description of the Related Art

### BACKGROUND ART

Conventionally, in the field of the endoscope, there has been a capsule medical device having an imaging function and a wireless communication function in a capsule-shaped casing formed into a size insertable in a digestive tract of a subject such as a patient. The capsule medical device moves in the digestive tract by peristalsis and the like after being swallowed from a mouth of the subject. In a period until the capsule endoscope is excreted from the subject since guided into the subject, the capsule endoscope sequentially captures images of an internal organ of the subject (hereinafter referred to as in-vivo images) and sequentially and wirelessly transmits the captured in-vivo images to a transmitting device outside of the subject.

Each in-vivo image captured by the capsule medical device is taken in an image display device through a receiving device. The image display device displays a still image or a moving image of each taken in-vivo image on a display. A user such as a doctor and a nurse observes the in-vivo images of the subject, which are displayed on the image display device, and inspects the internal organ of the subject through the observations of the in-vivo images.

On the other hand, recently there has been proposed a capsule medical device guidance system that guides the capsule medical device in the subject by a magnetic force (hereinafter referred to as magnetic induction). Generally, in the capsule medical device guidance system, the capsule medical device further includes a permanent magnet in a capsule-shaped casing, and the image display device displays a real-time in-vivo image sequentially captured by the capsule medical device in the subject. In the capsule medical device guidance system, a magnetic field is applied to the capsule medical device in the subject, and the capsule medical device in the subject is magnetically guided to a desired position by the magnetic force of the applied magnetic field. A user operates the magnetic induction of the capsule medical device with an operating unit of the capsule medical device guidance system while referring to the in-vivo image displayed on the image display device.

Some of the capsule endoscopes have specific gravity with which the capsule endoscopes can float in a liquid guided into the internal organ in order to observe the internal organ, such as a stomach and a large intestine, which has a relatively large space, and the capsule endoscopes sequentially capture the in-vivo images while floating in the liquid. Sometimes, the subject is to ingest a liquid that expands the internal organ (specifically, folds on an inner wall of the organ) and the capsule endoscope having the specific gravity smaller than that of the liquid in order to intensively inspect the internal organ, such as the stomach, which has the relatively large space (see, for example, Patent literature 1). In this case, the capsule endoscope sequentially captures the images of the internal organ expanded by the liquid while floating on a liquid level with a predetermined posture (for example, a longitudinal posture in which a center axis in a lengthwise direction of the capsule endoscope becomes substantially perpendicular to the liquid level). The capsule endoscope moves in a desired direction while floating on the liquid level of the internal organ, which allows the capsule endoscope to widely capture the images of the internal organ.
Patent literature 1: International Patent Application No. 2007/077922

Document US 2007/0221233 A1 describes a capsule endoscope including a drive unit for changing at least one of the position and posture of the casing in a liquid which is introduced in a subject. A control unit includes a magnetic field controller for controlling the magnetic field strength of a magnetic field generator. The magnetic field generator generates a magnetic field with the drive power controlled by the magnetic field controller and captures the capsule endoscope that floats in the surface of the liquid by its magnetic force. Next, the magnetic field controller controls the drive power to the magnetic field generator based on position/posture information of the capsule endoscope and controls the magnetic field strength to keep the capsule endoscope under the surface of the liquid by the magnetic field of the magnetic field generator.

### DISCLOSURE OF INVENTION

### PROBLEM TO BE SOLVED BY THE INVENTION

With capsule medical device guidance systems, conventionally, not only a magnetic field called as a uniform magnetic field but also a gradient magnetic field that has a substantially uniform magnetic field gradient are generated to move a capsule endoscope. However, a magnetic field generating unit cannot generate a gradient magnetic field that has a perfectly uniform magnetic field gradient in a space where a capsule endoscope is guided. As a result, because the generated magnetic field varies depending on the position in the space, in some cases, a micro force is generated and exerted on the capsule endoscope in an unintended direction or a force generated by the capsule endoscope changes. Therefore, if the capsule endoscope is on the inner wall of an organ or the liquid level, behavior of the capsule endoscope is stable, even when a force exerted on the capsule endoscope changes, due to a binding force by the inner wall of an organ or the liquid level; however, if the capsule endoscope is guided away from the inner wall of an organ or the liquid level into the liquid, because no binding force is exerted on the capsule endoscope, a change in a force exerted on the capsule endoscope makes behavior of the capsule endoscope instable. Therefore, with conventional systems, a problem occurs that the capsule endoscope cannot stop or moves in an unintended direction.

The present invention has been made in consideration of the abovementioned matters and it is an object of the present invention to provide a capsule medical device guidance system capable performing stable guidance of a capsule endoscope existing in liquid.

### MEANS FOR SOLVING PROBLEM

To solve the problem and achieve the object, a capsule medical device guidance system according to the appended claim 1 is provided.

Moreover, in the capsule medical device guidance system according to the present invention, a magnetic gradient of the magnetic field which is generated when the contact mode is selected and a magnetic gradient of the magnetic field which is generated when the away mode is selected are each a vertical magnetic gradient.

Moreover, the capsule medical device guidance system according to the present invention further includes a storage unit that stores therein information on control of the magnetic field generating unit. The operation input unit further includes a gradient instruction unit that issues an instruction regarding an optimum condition of the magnetic gradient of the magnetic field which is generated when the away mode is selected. The magnetic gradient of the magnetic field which is generated by the magnetic field generating unit when the gradient instruction unit issues the instruction, is stored as the optimum condition in the storage unit. The control unit causes the magnetic field generating unit to generate the magnetic gradient of the optimum condition stored in the storage unit when the away mode is selected.

Furthermore, in the capsule medical device guidance system according to the present invention, the magnetic field generating unit generates a magnetic gradient in a different direction from the direction of the magnetic gradient which is generated when each of the contact mode and the away mode is selected. The operation input unit further includes a magnetic field generating instruction unit that issues an instruction to generate the magnetic gradient in the different direction. The control unit causes the magnetic field generating unit to generate the magnetic gradient in the different direction while causing the magnetic field generating unit to generate the magnetic gradient which is generated when the contact mode and the away mode are each selected, when the magnetic field generating instruction unit issues the instruction to generate the magnetic gradient in the different direction.

Moreover, the capsule medical device guidance system according to the present invention further includes a behavior detector that detects a behavior of the capsule medical device. The control unit changes the magnetic gradient which is generated when the away mode is selected, in a stepwise manner, sets the optimum magnetic gradient based on a detection result of the behavior detector, and causes the magnetic field generating unit to generate the set optimum magnetic gradient.

Furthermore, in the capsule medical device guidance system according to the present invention, a balance between the buoyant force of the capsule medical device and the gravity of the capsule medical device is substantially established. The control unit causes the magnetic field generating unit to generate a first magnetic field having a first magnetic gradient that brings the capsule medical device into contact with the desired boundary surface when the selection unit selects the contact mode, and causes the magnetic field generating unit to generate a second magnetic field having a second magnetic gradient that moves the capsule medical device away from the desired boundary surface when the selection unit selects the away mode.

Moreover, in the capsule medical device guidance system according to the present invention, a direction of the first magnetic gradient is opposite to a direction of the second magnetic gradient.

Furthermore, in the capsule medical device guidance system according to the present invention, a total force of the buoyant force of the capsule medical device and the gravity of the capsule medical device is oriented toward the desired boundary surface side. The control unit causes the magnetic field generating unit to generate a first magnetic field having a first magnetic gradient that moves the capsule medical device away from the desired boundary surface when the selection unit selects the away mode, and causes the magnetic field generating unit to stop the generation of the first magnetic field when the selection unit selects the contact mode.

Moreover, in the capsule medical device guidance system according to the present invention, a direction of the first magnetic gradient is opposite to the direction oriented toward the desired boundary surface side.

Furthermore, in the capsule medical device guidance system according to the present invention, a total force of the buoyant force of the capsule medical device and the gravity of the capsule medical device is oriented toward a direction except the boundary surface side. The control unit causes the magnetic field generating unit to generate a first magnetic field having a first magnetic gradient that brings the capsule medical device into contact with the desired boundary surface when the selection unit selects the contact mode, and causes the magnetic field generating unit to stop the generation of at least the first magnetic field when the selection unit selects the away mode.

Moreover, in the capsule medical device guidance system according to the present invention, a direction of the first magnetic gradient is opposite to the direction oriented toward the desired boundary surface side.

Furthermore, in the capsule medical device guidance system according to the present invention, a direction of the first magnetic gradient is a vertical magnetic gradient.

### EFFECT OF THE INVENTION

A capsule medical device guidance system according to the present invention has a contact mode, in which the capsule medical device is brought into contact with a desired boundary surface in a plurality of boundary surfaces of a liquid in a subject, and an away mode, in which the capsule medical device is moved away from the desired boundary surface. When the contact mode is selected, a magnetic field generating unit is controlled such that a total force in the liquid of a buoyant force of the capsule medical device, a gravity of the capsule medical device, and a magnetic attraction force is oriented toward the desired boundary surface side. When the away mode is selected, the magnetic field generating unit is controlled such that the total force in the liquid of the buoyant force of the capsule medical device, the gravity of the capsule medical device, and the magnetic attraction force is oriented toward a direction other than the direction oriented toward the desired boundary surface side. Therefore, when behavior of the capsule endoscope becomes instable in the liquid, because the capsule endoscope can quickly be immobilized and stabilized in a reference plane, the capsule endoscope existing in liquid can stably be induced by a simple operation.

### BRIEF DESCRIPTION OF DRAWINGS

[FIG. 1] FIG. 1 is a schematic diagram illustrating an entire configuration of a capsule medical device guidance system according to a first embodiment.
[FIG. 2] FIG. 2 is a sectional schematic diagram illustrating an example of a configuration of a capsule endoscope illustrated in FIG. 1.
[FIG. 3] FIG. 3 is a block diagram illustrating a configuration of an operation input unit illustrated in FIG. 1.
[FIG. 4] FIG. 4 is a view illustrating an example of the operation input unit illustrated in FIG. 1 for the purpose of explaining magnetic induction in a liquid level area of a capsule medical device that can be operated by the operation input unit.
[FIG. 5] FIG. 5 is a view explaining movement of the capsule endoscope illustrated in FIG. 1.
[FIG. 6] FIG. 6 is a view explaining the movement of the capsule endoscope illustrated in FIG. 1.
[FIG. 7] FIG. 7 is a view explaining the movement of the capsule endoscope illustrated in FIG. 1.
[FIG. 8] FIG. 8 is a flowchart illustrating a procedure of capsule endoscope guidance process performed by the capsule medical device guidance system illustrated in FIG. 1.
[FIG. 9] FIG. 9 is a flowchart illustrating a procedure of away magnetic field applying process illustrated in FIG. 8.
[FIG. 10] FIG. 10 is a view explaining an example of the magnetic induction.
[FIG. 11] FIG. 11 is a view explaining another example of the magnetic induction.
[FIG. 12] FIG. 12 is a conceptual view explaining another example of the capsule endoscope illustrated in FIG. 1.
[FIG. 13] FIG. 13 is a view explaining the movement of the capsule endoscope illustrated in FIG. 12.
[FIG. 14] FIG. 14 is a conceptual view explaining another example of the capsule endoscope illustrated in FIG. 1.
[FIG. 15] FIG. 15 is a view explaining the movement of the capsule endoscope illustrated in FIG. 14.
[FIG. 16] FIG. 16 is a schematic diagram illustrating an entire configuration of a capsule medical device guidance system according to a second embodiment.
[FIG. 17] FIG. 17 is a block diagram illustrating a configuration of an operation input unit illustrated in FIG. 16.
[FIG. 18] FIG. 18 is a flowchart illustrating a procedure of capsule endoscope guidance process performed by the capsule medical device guidance system illustrated in FIG. 16.
[FIG. 19] FIG. 19 is a schematic diagram illustrating an entire configuration of a capsule medical device guidance system according to a modification of the second embodiment.
[FIG. 20] FIG. 20 is a view explaining a position detection process performed by a position detector illustrated in FIG. 19.
[FIG. 21] FIG. 21 is a view explaining the position detection process performed by the position detector illustrated in FIG. 19.
[FIG. 22] FIG. 22 is a flowchart illustrating a procedure of capsule endoscope guidance process performed by the capsule medical device guidance system illustrated in FIG. 19.

### BEST MODE(S) FOR CARRYING OUT THE INVENTION

Hereinafter, a capsule medical device guidance system according to an embodiment of the present invention will be described by taking a capsule medical device system, in which a capsule endoscope that is orally guided into a subject to float on a liquid accumulated in a stomach, a small intestine, a large intestine and the like of the subject is used as an in-vivo guidance device, as an example. However, the invention is not limited to the capsule endoscope. For example, various in-vivo guidance devices such as a monocular or binocular capsule endoscope that performs an imaging operation to capture the in-vivo image of the subject during movement in a body cavity from an esophagus of the subject to an anus can be used. The invention is not limited to the embodiments. In the drawings, the same component is denoted by the same numeral.

### (First Embodiment)

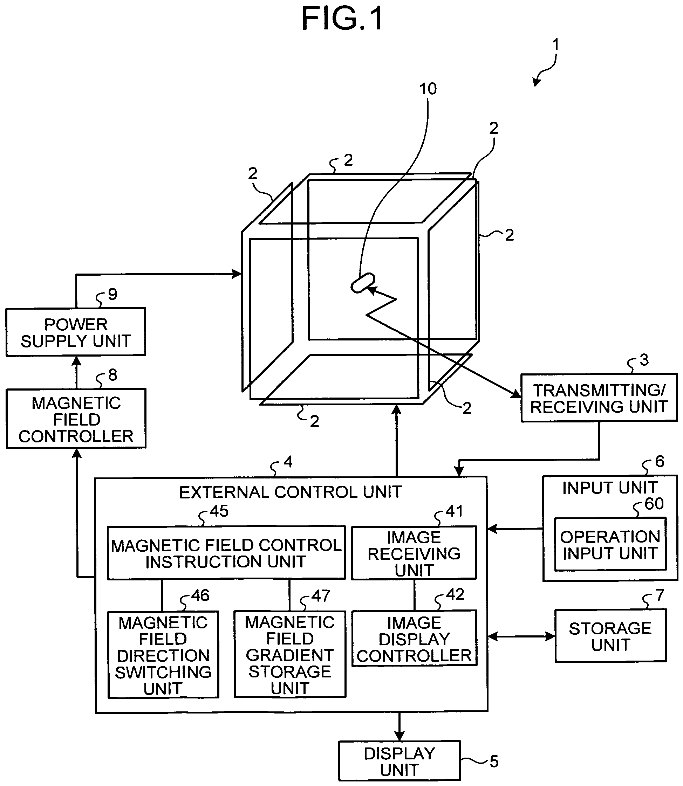

A first embodiment will be described. FIG. 1 is a schematic diagram illustrating an entire configuration of a capsule medical device guidance system according to a first embodiment of the invention. As illustrated in FIG. 1, a capsule medical device guidance system 1 of the first embodiment includes a capsule endoscope 10 that is of a capsule medical device, which is swallowed from a mouth of a subject and guided to a body cavity of the subject to conduct communication with an external device, a magnetic field generating unit 2 that is provided near the subject to be able to generate a three-dimensional magnetic field, a transmitting/receiving unit 3 that transmits an operation signal to the capsule endoscope 10 while conducting wireless communication with the capsule endoscope 10 to receive a radio signal including an image captured by the capsule endoscope 10, an external control unit 4 that controls each component of the capsule medical device guidance system 1, a display unit 5 that displays the image captured by the capsule endoscope 10, an input unit 6 that inputs pieces of instruction information, such as operation information for guiding magnetically the capsule endoscope 10, which issue instructions of various operations in the capsule medical device guidance system 1, to the external control unit 4, a storage unit 7 in which information on the image captured by the capsule endoscope 10 is stored, a magnetic field controller 8 that controls the magnetic field relating to the magnetic field generating unit 2, and a power supply unit 9 that supplies an electric power to the magnetic field generating unit 2 under the control of the magnetic field controller 8.

The transmitting/receiving unit 3 detects a position and an posture of the capsule endoscope 10 in the subject based on received electric field intensity of the signal transmitted from the capsule endoscope 10. Alternatively, a position detector may be provided in order to detect the position and the posture of the capsule endoscope 10. For example, a magnetic field generating unit or a magnetic field reflecting unit is provided in the capsule endoscope 10, plural magnetic field sensors are provided so as to surround the capsule endoscope 10 similarly to the magnetic field generating unit 2, and the position and the posture of the capsule endoscope 10 may be detected based on detection results of the magnetic field sensors.

The capsule endoscope 10 is a capsule medical device that captures the in-vivo image of the subject, and the capsule endoscope 10 is provided with an imaging function and a wireless communication function. The capsule endoscope 10 is guided into an internal organ of the subject by oral intake and the like. Then, in the subject, the capsule endoscope 10 moves in a digestive tract and finally excreted from the subject. In a period until the capsule endoscope 10 is excreted from the subject since guided into the subject, the capsule endoscope 10 sequentially captures the in-vivo images of the subject to sequentially and wirelessly transmit the captured in-vivo images to the transmitting/receiving unit 3. The capsule endoscope 10 is provided with a magnetic body such as a permanent magnet. The capsule endoscope 10 floats in a liquid guided to the internal organ of the subject (for example, an internal portion of the stomach) and is magnetically induced by the external magnetic field generating unit 2.

The magnetic field generating unit 2 is used for the magnetic induction of the capsule medical device in the subject. For example, the magnetic field generating unit 2 is constructed using plural coils and generates an induction magnetic field using the electric power supplied by the power supply unit 9. The magnetic field generating unit 2 applies the generated induction magnetic field to the magnetic body in the capsule endoscope 10 and magnetically traps the capsule endoscope 10 by action of the induction magnetic field. The magnetic field generating unit 2 controls a three-dimensional posture of the capsule endoscope 10 in the subject by changing a direction of the induction magnetic field acting on the capsule endoscope 10 in the subject.

The magnetic field generating unit 2 can generate a uniform-gradient magnetic field in addition to what is called a uniform magnetic field. The uniform-gradient magnetic field has a substantially uniform magnetic field gradient, and biases the permanent magnet of the capsule endoscope 10 in a direction in which a distribution of magnetic field intensity is inclined from coarseness to fineness. The magnetic field generating unit 2 generates the uniform-gradient magnetic field in which the distribution of the magnetic field intensity is inclined from coarseness to fineness in a direction in which the capsule endoscope 10 is biased, whereby biasing the permanent magnet to move the capsule endoscope 10 in the desired direction. The magnetic field generating unit 2 can also generate a peak magnetic field. The peak magnetic field has a peak of the magnetic field intensity in a direction perpendicular to a horizontal surface, and the capsule endoscope 10 can be constrained by attracting the permanent magnet to a peak position of the magnetic field intensity.

The transmitting/receiving unit 3 includes plural antennae and receives the in-vivo image of the subject from the capsule endoscope 10 through the antennae. The transmitting/receiving unit 3 sequentially receives the radio signals from the capsule endoscope 10 through the plural antennae. The transmitting/receiving unit 3 selects the antenna having the highest received electric field intensity from the plural antennae, and performs a demodulation process and the like to the radio signal received from the capsule endoscope 10 through the selected antenna. Therefore, the transmitting/receiving unit 3 extracts image data captured by the capsule endoscope 10 from the radio signal, namely, in-vivo image data of the subject. The transmitting/receiving unit 3 transmits an image signal including the extracted in-vivo image data to the external control unit 4.

The external control unit 4 controls operations of the magnetic field generating unit 2, the display unit 5, the storage unit 7, and the magnetic field controller 8, and controls input/output of the signal among the components. The external control unit 4 includes an image receiving unit 41 that sequentially acquires the in-vivo images sequentially received by the transmitting/receiving unit 3 and an image display controller 42 that displays the in-vivo images sequentially received by the transmitting/receiving unit 3 on the display unit 5 in real time. The external control unit 4 controls the storage unit 7 so as to store an in-vivo image group of the subject, which is acquired from the transmitting/receiving unit 3.

The external control unit 4 includes a magnetic field control instruction unit 45 that issues an instruction of a magnetic field generating condition to the magnetic field controller 8 in order to induce the capsule endoscope 10 according to operation information input from the input unit 6, a magnetic field direction switching unit 46 that switches a gradient direction of the uniform-gradient magnetic field generated by the magnetic field generating unit 2, and a magnetic field gradient storage unit 47 in which the magnetic field generating condition including the gradient direction of the uniform-gradient magnetic field generated by the magnetic field generating unit 2 is stored. In the case that the operation information on the capsule endoscope 10 is input by the input unit 6, the magnetic field control instruction unit 45 issues an instruction to generate the magnetic field to the magnetic field controller 8 according to a magnetic induction direction and a magnetic induction position, which are assigned by the input operation information.

The display unit 5 is constructed using various displays such as a liquid crystal display, and displays various pieces of information in response to the instruction of the external control unit 4. Specifically, for example, the display unit 5 displays the in-vivo image group of the subject captured by the capsule endoscope 10 under the control of the image display controller 42 of the external control unit 4. The display unit 5 also displays a reduced image of the in-vivo image, which is selected or marked from the in-vivo image group by the input operation of the input unit 6, and patient information and inspection information on the subject and the like.

The input unit 6 is constructed using an input device such as a keyboard and a mouse, and inputs various pieces of information to the external control unit 4 in response to the input operation performed by an operator such as a doctor. Examples of the various pieces of information input to the external control unit 4 by the input unit 6 include such as the instruction information for issuing the instruction to the external control unit 4, the patient information and the inspection information on the subject. The patient information on the subject is identification information, such as patient name, patient ID, date of birth, sex, and age, which identifies the subject. The inspection information on the subject is identification information, such as inspection ID and inspection date, which identifies the inspection guiding the capsule endoscope 10 into the digestive tract of the subject to observe the inside of the digestive tract. The input unit 6 inputs the operation information in order to operate the magnetic induction of the capsule endoscope 10 by the magnetic field generating unit 2.

The input unit 6 includes an operation input unit 60 that inputs the pieces of operation information, such as the magnetic induction direction and the magnetic induction position of the capsule endoscope 10 that is of the magnetic induction operation target, to the external control unit 4 in order to magnetically induce the capsule endoscope 10. The operation input unit 60 includes a joystick, various buttons, and various switches, and the operator operates the joystick and the like to input the operation information to the external control unit 4.

The storage unit 7 is constructed using a storage medium such as a flash memory and a hard disk, in which the information is stored in a rewritable manner. The storage unit 7 stores the various pieces of information therein in response to the storage instruction of the external control unit 4, and the storage unit 7 transmits the information that the external control unit 4 issues the instruction to read from the stored various pieces of information to the external control unit 4. Examples of the various pieces of information stored in the storage unit 7 include the image data of the in-vivo image group of the subject, which is captured by the capsule endoscope 10, the data of the in-vivo image selected from the in-vivo images displayed on the display unit 5 by the input operation of the input unit 6, and the input information, such as the patient information on the subject, which is input by the input unit 6, and the like.

The magnetic field controller 8 controls a current-supplying amount of the power supply unit 9 to the magnetic field generating unit 2 based on the instruction information instructed by the external control unit 4, and the magnetic field controller 8 controls the magnetic field generating unit 2 through the control of the power supply unit 9 such that the induction magnetic field necessary for the magnetic induction of the capsule endoscope 10 is generated according to the magnetic induction direction and the magnetic induction position based on the operation information on the capsule endoscope 10.

The power supply unit 9 supplies the electric power (for example, alternating current) necessary to generate the induction magnetic field to the magnetic field generating unit 2 based on the control of the external control unit 4 and the magnetic field controller 8. In this case, the power supply unit 9 properly supplies the necessary electric power to each of the plural coils included in the magnetic field generating unit 2. The magnetic field direction and the magnetic field intensity of the induction magnetic field generated by the magnetic field generating unit 2 is controlled by the current-supplying amount from the power supply unit 9 to each coil of the magnetic field generating unit 2.

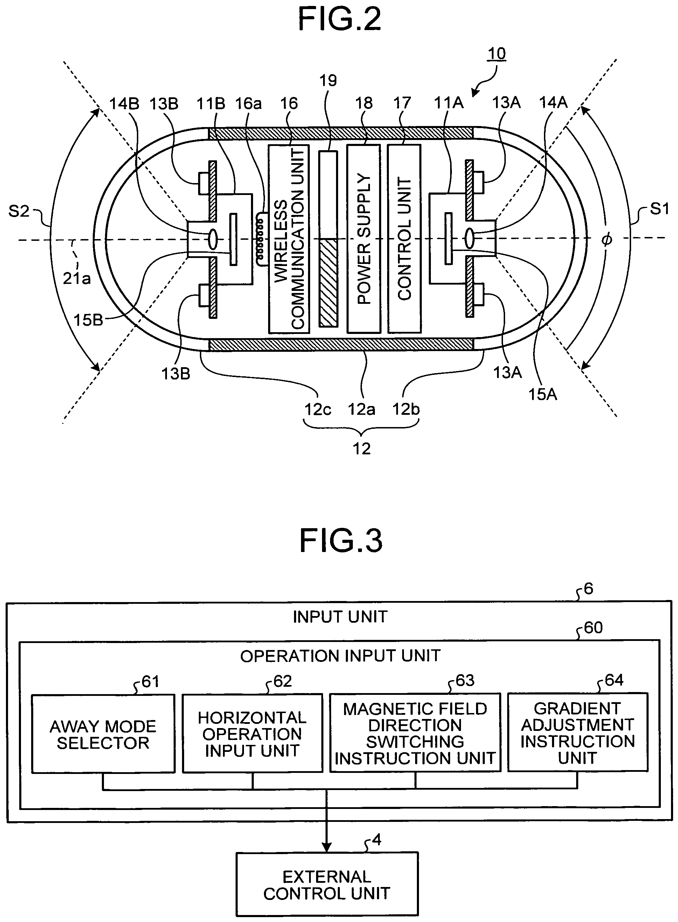

The capsule endoscope 10 will be described below. FIG. 2 is a sectional schematic diagram illustrating an example of a configuration of a capsule endoscope illustrated in FIG. 1. As illustrated in FIG. 2, the capsule endoscope 10 includes a capsule-shaped casing 12 that is of an outer package formed in to a size easily guided into the internal organ of the subject and imaging units 11A and 11B that capture the image of the subject in imaging directions different from each other. The capsule endoscope 10 includes a wireless communication unit 16 that wirelessly transmits the images captured by the imaging units 11A and 11B to the outside, a control unit 17 that controls each component of the capsule endoscope 10, and a power supply 18 that supplies the electric power to each component of the capsule endoscope 10. The capsule endoscope 10 includes a permanent magnet 19 that enables the magnetic induction by the magnetic field generating unit 2.

The capsule-shaped casing 12 is an outer casing formed in to a size easily guided into the internal organ of the subject, and is constructed by closing opening ends on both sides of a cylindrical casing 12a with a dome-shaped casings 12b and 12c. The dome-shaped casings 12b and 12c are dome-shaped optical members that are transparent to light having a predetermined wavelength band such as visible light. The cylindrical casing 12a is a colored casing that is substantially opaque to the visible light. As illustrated in FIG. 2, the capsule-shaped casing 12 including the cylindrical casing 12a and the dome-shaped casings 12b and 12c includes the imaging units 11A and 11B, the wireless communication unit 16, the control unit 17, the power supply 18, and the permanent magnet 19 in a liquid-tight manner.

The imaging units 11A and 11B capture the images in the directions different from each other. Specifically, the imaging unit 11A includes an illumination unit 13A such as an LED, an optical system 14A such as a condenser lens, and an imaging element 15A such as a CMOS image sensor and a CCD. The illumination unit 13A emits illumination light such as white light to an imaging visual field S1 of the imaging element 15A to illuminate the subject (for example, the inner wall of the internal organ on the side of the imaging visual field S1 in the subject) in the imaging visual field S1 over the dome-shaped casing 12b. The optical system 14A condenses the light reflected from the imaging visual field S1 onto an imaging surface of the imaging element 15A, and forms the subject image in the imaging visual field S1 on the imaging surface of the imaging element 15A. The imaging element 15A receives the light reflected from the imaging visual field S1 through the imaging surface, performs a photoelectric conversion process to the received light signal, and captures the subject image in the imaging visual field S1, namely, the in-vivo image of the subject. The imaging unit 11B includes an illumination unit 13B such as an LED, an optical system 14B such as a condenser lens, and an imaging element 15B such as a CMOS image sensor and a CCD. The illumination unit 13B emits the illumination light such as the white light to an imaging visual field S2 of the imaging element 15B to illuminate the subject (for example, the inner wall of the internal organ on the side of the imaging visual field S2 in the subject) in the imaging visual field S2 over the dome-shaped casing 12c. The optical system 14B condenses the light reflected from the imaging visual field S2 onto an imaging surface of the imaging element 15B, and forms the subject image in the imaging visual field S2 on the imaging surface of the imaging element 15B. The imaging element 15B receives the light reflected from the imaging visual field S2 through the imaging surface, performs the photoelectric conversion process to the received light signal, and captures the subject image in the imaging visual field S2, namely, the in-vivo image of the subject.

In the case that the capsule endoscope 10 is a binocular type capsule medical device that captures front and rear images in a direction of a long axis 21a as illustrated in FIG. 2, optical axes of the imaging units 11A and 11B are substantially parallel or aligned with the long axis 21a that is of a center axis in the lengthwise direction of the capsule-shaped casing 12. The directions of the imaging visual fields S1 and S2 of the imaging units 11A and 11B, namely, imaging directions of the imaging units 11A and 11B are opposite to each other.

The wireless communication unit 16 includes an antenna 16a, and sequentially and wirelessly transmits the images captured by the imaging units 11A and 11B to the outside through the antenna 16a. Specifically, the wireless communication unit 16 acquires the image signal of the in-vivo image of the subject, which is captured by the imaging unit 11A or 11B, from the control unit 17, performs a modulation process to the acquired image signal, and generates the radio signal in which the image signal is modulated. The wireless communication unit 16 transmits the radio signal to the transmitting/receiving unit 3 through the antenna 16a.

The control unit 17 controls the operations of the imaging units 11A and 11B and the wireless communication unit 16, which are of the components of the capsule endoscope 10, and controls the input/output of the signal among the components. Specifically, the control unit 17 causes the imaging element 15A to capture the image of the subject in the imaging visual field S1 illuminated by the illumination unit 13A, and causes the imaging element 15B to capture the image of the subject in the imaging visual field S2 illuminated by the illumination unit 13B. The control unit 17 has a signal processing function of generating the image signal. The control unit 17 acquires the in-vivo image data in the imaging visual field S1 from the imaging element 15A, performs predetermined signal processing to the in-vivo image data in each case, and generates the image signal including the in-vivo image data in the imaging visual field S1. Similarly, the control unit 17 acquires the in-vivo image data in the imaging visual field S2 from the imaging element 15B, performs predetermined signal processing to the in-vivo image data in each case, and generates the image signal including the in-vivo image data in the imaging visual field S2. The control unit 17 controls the wireless communication unit 16 such that the image signals are sequentially and wirelessly transmitted to the outside in time series.

The power supply 18 is an electric accumulator such as a button battery and a capacitor, and is constructed by the electric accumulator and a switch such as a magnetic switch. The power supply 18 switches between an on-state and an off-state of the power supply by the magnetic field applied from the outside, and properly supplies the electric power of the electric accumulator to each component (the imaging units 11A and 11B, the wireless communication unit 16, and the control unit 17) of the capsule endoscope 10 in the case of the off-state. The power supply 18 stops the supply of the electric power to each component of the capsule endoscope 10 in the case of the off-state.

The permanent magnet 19 enables the magnetic field generating unit 2 to perform the magnetic induction of the capsule endoscope 10. The permanent magnet 19 is fixedly disposed in the capsule-shaped casing 12 while relatively fixed to the imaging units 11A and 11B. In this case, the permanent magnet 19 is magnetized in a well-known direction relatively fixed to vertical directions of the imaging surfaces of the imaging elements 15A and 15B.

In the first embodiment, a contact mode and an away mode are set while correlated with each other as a guidance mode of the capsule endoscope 10. The contact mode is one, in which the capsule endoscope 10 is brought into contact with a desired boundary surface in plural boundary surfaces of a liquid in the subject while pressed the desired boundary surface. The away mode is one, in which the capsule endoscope 10 in contact with the desired boundary surface is moved away from the desired boundary surface.

For example, in the case that the capsule endoscope 10 is located in the stomach, and in the case that the magnetic induction of the capsule endoscope 10 is performed based on the liquid level that is of the upper boundary surface of the liquid in the stomach, the contact mode corresponds to the case in which the capsule endoscope 10 is retained while brought into contact with the liquid level, and the away mode corresponds to the case in which the capsule endoscope 10 is moved away from the liquid level and guided downward into the liquid. In the case that the magnetic induction of the capsule endoscope 10 is performed based on a stomach wall in a bottom portion that is of the lower boundary surface of the liquid in the stomach, the contact mode corresponds to the case in which the capsule endoscope 10 is retained while brought into contact with the stomach wall, and the away mode corresponds to the case in which the capsule endoscope 10 is moved away from the stomach wall and guided upward into the liquid. The magnetic induction corresponding to each mode is implemented such that the magnetic field generating unit 2 generates the uniform-gradient magnetic field. The gradient direction of the uniform-gradient magnetic field generated in each of the contact mode and the away mode is switched based on whether the capsule endoscope 10 is located in the upper portion or the lower portion in the stomach. The contact mode or the away mode is implemented by inputting selection information indicating whether the contact mode or the away mode is selected by the operation input unit 60 to the external control unit 4.

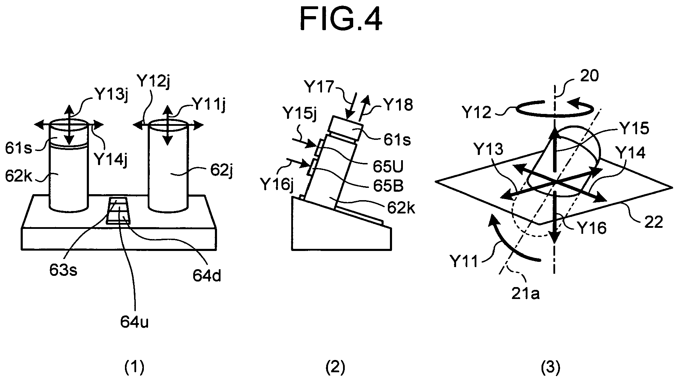

A configuration of the operation input unit 60 will be described. FIG. 3 is a block diagram illustrating the configuration of the operation input unit 60 illustrated in FIG. 1, and FIG. 4 is a view illustrating an example of the operation input unit 60 illustrated in FIG. 1 and explaining the magnetic induction in the liquid level area of the capsule medical device that can be operated by the operation input unit 60. FIG. 4(1) is a front view of the operation input unit 60, FIG. 4(2) is a left side view of the operation input unit 60, and FIG. 4(3) is a view illustrating operation contents of the capsule endoscope 10 that is instructed by the operation of each component of the operation input unit 60. As illustrated in FIGS. 3, 4(1), and 4(2), the operation input unit 60 includes an away/contact mode selector 61, a horizontal operation input unit 62, a magnetic field direction switching instruction unit 63, and a gradient adjustment instruction unit 64.

The away/contact mode selector 61 inputs the selection information selecting one of the away mode and the contact mode to the external control unit 4. The away/contact mode selector 61 includes a away mode button 61s provided on the top of a joystick 62k. The away mode button 61s inputs the selection information selecting the away mode to the external control unit 4 by pressing the away mode button 61s as illustrated by an arrow Y17 of FIG. 4(2). The away mode button 61s inputs the selection information selecting the contact mode to the external control unit 4 by releasing the pressing the away mode button 61s as illustrated by an arrow Y18 of FIG. 4(2).

The horizontal operation input unit 62 inputs the operation information on the magnetic induction in the horizontal direction of the capsule endoscope 10 by the magnetic field generating unit 2 to the external control unit 4. For example, the horizontal operation input unit 62 includes two joysticks 62j and 62k. The joysticks 62j and 62k can be tilted in the vertical and horizontal directions, and the operation information is input to the external control unit 4 in order to three-dimensionally operate the magnetic induction of the capsule endoscope 10 by the magnetic field generating unit 2.

The magnetic field direction switching instruction unit 63 performs the magnetic induction of the capsule endoscope 10 based on the liquid level that is of the boundary surface in the upper portion of the liquid in the stomach or the stomach wall of the bottom that is of the boundary surface in the lower portion of the liquid in the stomach, thereby inputting the instruction information issuing the instruction to switch the gradient direction of the uniform-gradient magnetic field generated in the contact mode and the away mode to the external control unit 4. The magnetic field direction switching unit 46 switches the gradient direction of the uniform-gradient magnetic field generated in each of the contact mode and the away mode to the predetermined direction in the case that the magnetic field direction switching instruction unit 63 issues the instruction to switch the gradient direction of the uniform-gradient magnetic field generated in each of the contact mode and the away mode.

The magnetic field direction switching instruction unit 63 includes an up/down mode changeover switch 63s. For example, an up mode in which the magnetic induction of the capsule endoscope 10 is performed based on the liquid level that is of the boundary surface in the upper portion of the liquid in the stomach or the upper stomach wall and a down mode in which the magnetic induction of the capsule endoscope 10 is performed based on the stomach wall of the bottom that is of the boundary surface in the lower portion of the liquid in the stomach are set. For example, the up/down mode changeover switch 63s is pressed to perform the display indicating that the up mode is selected, and outputs the instruction information issuing the instruction to switch the gradient direction of the uniform-gradient magnetic field generated in each of the contact mode and the away mode to the direction corresponding to the up mode to the external control unit 4. For example, the up/down mode changeover switch 63s performs the display indicating that the down mode is selected by releasing the pressing, and outputs the instruction information issuing the instruction to switch the gradient direction of the uniform-gradient magnetic field generated in each of the contact mode and the away mode to the direction corresponding to the down mode to the external control unit 4.

The gradient adjustment instruction unit 64 inputs the instruction information issuing the instruction to adjust the gradient of the uniform-gradient magnetic field generated in the away mode to the external control unit 4. The gradient adjustment instruction unit 64 includes a gradient up button 64u and a gradient down button 64d. The gradient up button 64u is pressed to input the instruction information indicating that the gradient of the uniform-gradient magnetic field is incremented by one stage to the external control unit 4. The gradient down button 64d is pressed to input the instruction information indicating that the gradient of the uniform-gradient magnetic field is decremented by one stage to the external control unit 4.

A behavior of the capsule endoscope 10 corresponding to the tilt operations of the joysticks 62j and 62k will be described. As illustrated in FIG. 4(1), the tilt in the vertical direction illustrated by an arrow 11j of the joystick 62j corresponds to a direction of a tilting behavior in which a front end of the capsule endoscope 10 oscillates so as to pass through a vertical axis 20 as illustrated by an arrow Y11 of FIG. 4(3). In the case that the operation input unit 60 inputs the operation information corresponding to the tilt operation of the arrow 11j of the joystick 62j to the external control unit 4, based on the operation information, the magnetic field control instruction unit 45 calculates an induction direction of the front end of the capsule endoscope 10 on an absolute coordinate system according to the tilt direction of the joystick 62j, and calculates an induction speed according to the tilt operation of the joystick 62j. The magnetic field control instruction unit 45 selects the peak magnetic field, which is switched by the magnetic field direction switching unit 46, as the applied magnetic field, and changes an angle formed between the orientation corresponding to the calculated induction direction and the vertical axis 20 in a vertical plane including the vertical axis 20 and a long axis 21a of the capsule endoscope 10.

As illustrated in FIG. 4(1), the tilt in the horizontal direction illustrated by an arrow 12j of the joystick 62j corresponds to a direction of a rotation behavior in which the capsule endoscope 10 rotates about the vertical axis 20 as illustrated by an arrow Y12 of FIG. 4(3). In the case that the operation input unit 60 inputs the operation information corresponding to the tilt operation of the arrow Y12j of the joystick 62j to the external control unit 4, based on the operation information, the magnetic field control instruction unit 45 calculates the induction direction of the front end of the capsule endoscope 10 on the absolute coordinate system according to the tilt direction of the joystick 62j, calculates the induction speed according to the tilt operation of the joystick 62j, causes the magnetic field generating unit 2 to generate the peak magnetic field having the orientation corresponding to the calculated induction direction, and rotates the orientation of the peak magnetic field about the vertical axis 20 at the calculated induction speed.

As illustrated in FIG. 4(1), the tilt in the vertical direction illustrated by an arrow Y13j of the joystick 62k corresponds to a direction of a horizontal backward behavior or a horizontal forward behavior in which the capsule endoscope 10 travels in a direction, in which the long axis 21a of the capsule endoscope 10 is projected to a horizontal surface 22, as illustrated by an arrow Y13 of FIG. 4(3). In the case that the operation input unit 60 inputs the operation information corresponding to the tilt operation of the arrow Y13j of the joystick 62k to the external control unit 4, based on the operation information, the magnetic field control instruction unit 45 calculates the induction direction and the induction position of the front end of the capsule endoscope 10 on the absolute coordinate system according to the tilt direction of the joystick 62k, calculates the induction speed according to the tilt operation of the joystick 62k, causes the magnetic field generating unit 2 to generate the peak magnetic field having the orientation corresponding to the calculated induction direction, and moves the peak of the peak magnetic field to the induction position at the calculated induction speed.

As illustrated in FIG. 4(1), the tilt in the horizontal direction illustrated by an arrow Y14j of the joystick 62k corresponds to a direction of a horizontal right behavior or a horizontal left behavior in which the capsule endoscope 10 travels perpendicular to a direction, in which the long axis 21a of the capsule endoscope 10 is projected to a horizontal surface 22, as illustrated by an arrow Y14 of FIG. 4(3). In the case that the operation input unit 60 inputs the operation information corresponding to the tilt operation of an arrow 14j of the joystick 62k to the external control unit 4, based on the operation information, the magnetic field control instruction unit 45 calculates the induction direction and the induction position of the front end of the capsule endoscope 10 on the absolute coordinate system according to the tilt direction of the joystick 62k, calculates the induction speed according to the tilt operation of the joystick 62k, causes the magnetic field generating unit 2 to generate the peak magnetic field having the orientation corresponding to the calculated induction direction, and moves the peak of the peak magnetic field to the induction position at the calculated induction speed.

An up button 65U and a down button 65B are provided in a rear surface of the joystick 62k. An up behavior in which the capsule endoscope 10 travels upward along the vertical axis 20 as illustrated by an arrow Y15 of FIG. 4(3) is instructed in the case that the up button 65U is pressed as illustrated by an arrow Y15j of FIG. 4(2). A down behavior in which the capsule endoscope 10 travels downward along the vertical axis 20 as illustrated by an arrow Y16 of FIG. 4(3) is instructed in the case that the down button 65B is pressed as illustrated by an arrow Y16j of FIG. 4(2). In the case that the operation input unit 60 inputs the operation information corresponding to the pressing operation to press the up button 65U or the down button 65B in the direction of the arrow Y15j or Y16j to the external control unit 4, based on the operation information, the magnetic field control instruction unit 45 calculates the behavior direction of the front end of the capsule endoscope 10 on the absolute coordinate system according to which button is pressed, and causes the magnetic field generating unit 2 to generate the uniform-gradient magnetic field having the gradient along the vertical axis 20 according to the calculated behavior direction. In the case that the up button 65U is pressed, the magnetic field generating unit 2 generates the uniform-gradient magnetic field having the gradient that becomes fine toward the upward direction of the vertical axis 20, thereby moving the capsule endoscope 10 as illustrated by the arrow Y15. In the case that the down button 65B is pressed, the magnetic field generating unit 2 generates the uniform-gradient magnetic field having the gradient that becomes fine toward the downward direction of the vertical axis 20, thereby moving the capsule endoscope 10 as illustrated by the arrow Y16.

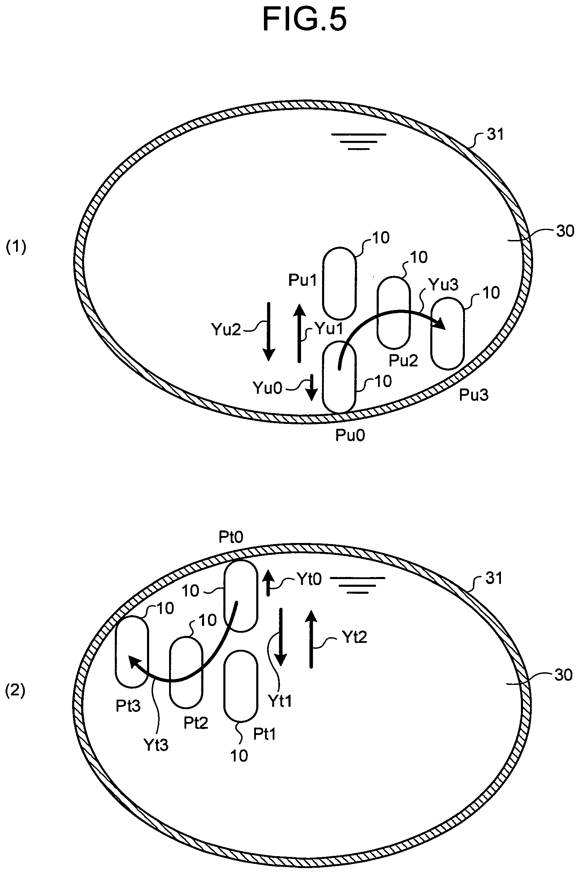

How the capsule endoscope 10 moves by the operation of the operation input unit 60 will specifically be described with reference to FIG. 5. FIG. 5 illustrates an example in which the capsule endoscope 10 is guided in the stomach to which a liquid 30 is introduced.

The case in which the operator operates the up/down mode changeover switch 63s to select the down mode will be described with reference to FIG. 5(1). This case corresponds to the case in which the instruction information issuing the instruction to select the up mode is input from the operation input unit 60. In the case of the down mode, the magnetic induction of the capsule endoscope 10 is performed based on a stomach wall 31 of the bottom in the boundary surface of the liquid 30.

In the case of the down mode, the contact mode corresponds to the mode in which the capsule endoscope 10 is retained while pressed against the stomach wall 31 of the bottom. Therefore, in the contact mode, it is necessary for the magnetic field control instruction unit 45 to cause the magnetic field generating unit 2 to generate the magnetic field having a magnetic attraction force in the downward direction of the vertical axis. The magnetic field direction switching unit 46 issues the instruction to switch the gradient direction of the uniform-gradient magnetic field in the contact mode to the downward direction of the vertical axis to the magnetic field control instruction unit 45.

In the case of the down mode, the away mode corresponds to the mode in which the capsule endoscope 10 is moved upward from the stomach wall 31 of the bottom. Therefore, in the away mode, it is necessary for the magnetic field control instruction unit 45 to cause the magnetic field generating unit 2 to generate the magnetic field having the magnetic attraction force in the upward direction of the vertical axis. The magnetic field direction switching unit 46 issues the instruction to switch the gradient direction of the uniform-gradient magnetic field in the away mode to the upward direction of the vertical axis to the magnetic field control instruction unit 45. In the case of the down mode, the gradient direction of the uniform-gradient magnetic field in the contact mode and the gradient direction of the uniform-gradient magnetic field in the away mode are set in opposite directions to each other.

Accordingly, in the case that the down mode is selected, in order to retain the capsule endoscope 10 at a position Pu0 of the bottom, the magnetic field control instruction unit 45 causes the magnetic field generating unit 2 to generate the uniform-gradient magnetic field having the vertically downward gradient in which the capsule endoscope 10 is pressed at the position Pu0 of the bottom. As a result, the capsule endoscope 10 retains a stable rest state as illustrated by an arrow Yu0 because the capsule endoscope 10 is pressed at the position Pu0 against the stomach wall 31 of the bottom that becomes the reference.

In the case that the operator presses the away mode button 61s, in order to upwardly move the capsule endoscope 10 away from the position Pu0 of the bottom, the magnetic field control instruction unit 45 causes the magnetic field generating unit 2 to generate the uniform-gradient magnetic field having the vertically upward gradient in which the capsule endoscope 10 is moved upward. As a result, the capsule endoscope 10 floats from the position Pu0 of the stomach wall 31 and is moved to a position Pu1 as illustrated by an arrow Yu1. In this case, during instructing the away mode, the magnetic field control instruction unit 45 may store the magnetic field generating condition of the contact mode before the away mode is switched.

In the case that the operator releases the pressing of the away mode button 61s, in order to correspond to the case in which the contact mode is selected, the magnetic field control instruction unit 45 causes the magnetic field generating unit 2 to generate the uniform-gradient magnetic field having the vertically downward gradient identical to that before the away mode is selected, and stabilizes the capsule endoscope 10 by pressing the capsule endoscope 10 against the stomach wall 31 of the bottom at the position Pu0 that becomes the reference.

The case in which the tilt operation of the joystick 62k is performed to issue the instruction of the horizontal movement while the operator presses the away mode button 61s will be described. In this case, the magnetic field control instruction unit 45 causes the magnetic field generating unit 2 to generate the uniform-gradient magnetic field having the magnetic gradient in the direction corresponding to the direction of the instruction of the horizontal movement while causing the magnetic field generating unit 2 to generate the uniform-gradient magnetic field having the vertically upward gradient corresponding to the away mode. As a result, the capsule endoscope 10 moves horizontally in the liquid from the position Pu0 to a position Pu3 through a position Pu2 while jumping obliquely upward as illustrated by an arrow Yu3.

The case in which the operator operates the up/down mode changeover switch 63s to switch from the down mode to the up mode will be described with reference to FIG. 5(2). This case corresponds to the case in which the instruction information issuing the instruction to select the up mode is input from the operation input unit 60. In the case of the up mode, the magnetic induction of the capsule endoscope 10 is performed based on the upper stomach wall 31 in the boundary surface of the liquid 30 or the liquid level.

In the case of the up mode, the contact mode corresponds to the mode in which the capsule endoscope 10 is retained while pressed against the upper stomach wall 31 or the liquid level. Therefore, in the contact mode, it is necessary for the magnetic field control instruction unit 45 to cause the magnetic field generating unit 2 to generate the magnetic field having the magnetic attraction force in the upward direction of the vertical axis. The magnetic field direction switching unit 46 issues the instruction to switch the gradient direction of the uniform-gradient magnetic field in the contact mode to the upward direction of the vertical axis to the magnetic field control instruction unit 45.

In the case of the up mode, the away mode corresponds to the mode in which the capsule endoscope 10 is moved downward from the upper stomach wall 31 or the liquid level. Therefore, in the away mode, it is necessary for the magnetic field control instruction unit 45 to cause the magnetic field generating unit 2 to generate the magnetic field having the magnetic attraction force in the downward direction of the vertical axis. The magnetic field direction switching unit 46 issues the instruction to switch the gradient direction of the uniform-gradient magnetic field in the away mode to the downward direction of the vertical axis to the magnetic field control instruction unit 45.

In the case of the up mode, similarly to the down mode, the gradient direction of the uniform-gradient magnetic field in the contact mode and the gradient direction of the uniform-gradient magnetic field in the away mode are set in opposite directions to each other. The magnetic field direction switching unit 46 switches between the gradient direction of the uniform-gradient magnetic field in the contact mode and the gradient direction of the uniform-gradient magnetic field in the away mode such that the gradient direction of the uniform-gradient magnetic field in the contact mode and the gradient direction of the uniform-gradient magnetic field in the away mode are inverted by switching from the up mode to the down mode. The switching from the down mode to the up mode means that the reference plane of the magnetic induction of the capsule endoscope 10 is switched from the lower stomach wall 31 to the upper stomach wall 31 or the liquid level. The up/down mode changeover switch 63s is switched from the up mode to the down mode to issue the instruction to invert the gradient direction of the uniform-gradient magnetic field in the contact mode and the gradient direction of the uniform-gradient magnetic field in the away mode.

Accordingly, in the case that the up mode is selected from the down mode, in order to retain the capsule endoscope 10 at a position Pt0 of the upper stomach wall 31, the magnetic field control instruction unit 45 causes the magnetic field generating unit 2 to generate the uniform-gradient magnetic field having the vertically upward gradient in which the capsule endoscope 10 is pressed at the position Pt0 of the upper stomach wall 31. As a result, the capsule endoscope 10 retains the stable rest state as illustrated by an arrow Yt0 because the capsule endoscope 10 is pressed at the position Pt0 against the upper stomach wall 31 that becomes the reference.

In the case that the operator presses the away mode button 61s, in order to downwardly move the capsule endoscope 10 away from the position Pt0 of the upper stomach wall 31, the magnetic field control instruction unit 45 causes the magnetic field generating unit 2 to generate the uniform-gradient magnetic field having the vertically downward gradient in which the capsule endoscope 10 is moved downward. As a result, the capsule endoscope 10 sinks in the liquid 30 from the position Pt0 of the stomach wall 31 and is moved to a position Pt1 as illustrated by an arrow Yt1.

In the case that the operator releases the pressing of the away mode button 61s, in order to correspond to the case in which the contact mode is selected, the magnetic field control instruction unit 45 causes the magnetic field generating unit 2 to generate the uniform-gradient magnetic field having the vertically upward gradient, and stabilizes the capsule endoscope 10 by pressing the capsule endoscope 10 against the upper stomach wall 31 at the position Pt0 that becomes the reference.

The case in which the tilt operation of the joystick 62k is performed to issue the instruction of the horizontal movement while the operator presses the away mode button 61s will be described. In this case, the magnetic field control instruction unit 45 causes the magnetic field generating unit 2 to generate the uniform-gradient magnetic field having the magnetic gradient in the direction corresponding to the direction of the instruction of the horizontal movement while causing the magnetic field generating unit 2 to generate the uniform-gradient magnetic field having the vertically downward gradient corresponding to the away mode. As a result, the capsule endoscope 10 moves horizontally in the liquid from the position Pt0 to a position Pt3 through a position Pt2 while jumping obliquely downward as illustrated by an arrow Yt3.

In the case that the operator operates the up/down mode changeover switch 63s to switch from the up mode to the down mode, as described above, the magnetic induction of the capsule endoscope 10 is performed based on the stomach wall 31 of the bottom in the boundary surface of the liquid. Therefore, the magnetic field direction switching unit 46 switches between the gradient direction of the uniform-gradient magnetic field in the contact mode and the gradient direction of the uniform-gradient magnetic field in the away mode such that the gradient direction of the uniform-gradient magnetic field in the contact mode and the gradient direction of the uniform-gradient magnetic field in the away mode are inverted, and the magnetic field generating unit 2 generates the uniform-gradient magnetic field having the gradient in the direction corresponding to each mode.

Only the operator switches the mode corresponding to the desired boundary surface using the up/down mode changeover switch 63s according to which boundary surface in the plural boundary surfaces of the liquid is set to the reference during the magnetic induction of the capsule endoscope 10, thereby automatically switching the gradients of the uniform-gradient magnetic fields corresponding to the contact mode and the away mode to the direction corresponding to the desired boundary surface. Therefore, it is not necessary for the operator to switch the magnetic gradient. Additionally, the capsule endoscope 10 can automatically and stably be retained in the desired boundary surface such that only the operator switches the up/down mode changeover switch 63s to the desired mode. Therefore, it is not necessary for the operator to perform movement operation of the capsule endoscope 10.

In the up mode or the down mode, which is selected by the operator, in the case that the operator performs zoom back of the imaging target located in the boundary surface, the operator presses the away mode button 61s only for a time the zoom back is performed. Only the joystick 62k is laid in the desired direction while away mode button 61s is pressed, which allows the capsule endoscope 10 to jump and move in the liquid. The operator can issue the instructions of various operations of the capsule endoscope 10 by the simple operation.

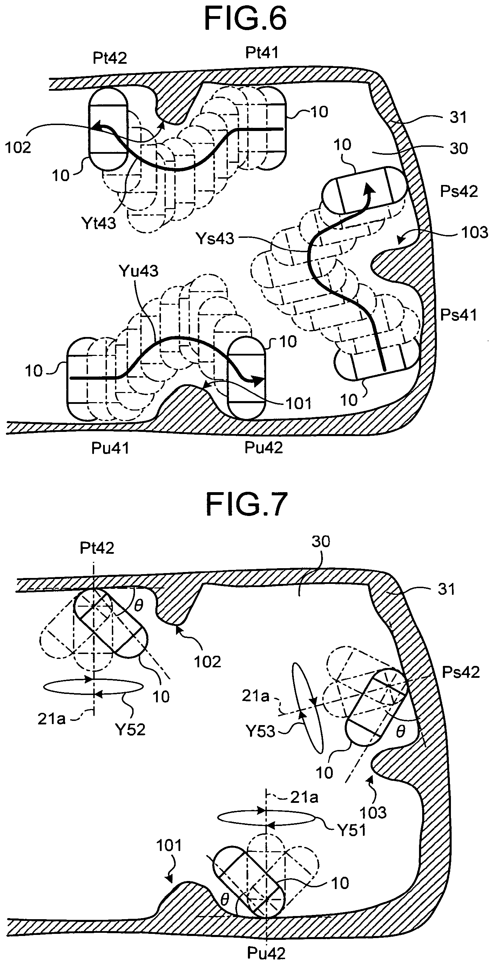

For example, as illustrated in FIG. 6, in the case that the capsule endoscope 10 is guided upright along the stomach wall 31 of the bottom in order to observe the interior portion of the upper stomach, the operator may operate the joysticks 62j and 62k without pressing the away mode button 61s while selecting the down mode. As a result, the contact mode is implemented, and the capsule endoscope 10 moves in the instructed induction direction while contacting with the stomach wall 31 of the bottom. In the case that a projection 101 such as a plica and a polyp which becomes an induction obstacle of the capsule endoscope 10 is discovered at a position Pu41 in the induction direction, it is necessary for the capsule endoscope 10 to jump over the projection 101 in order to continue the observation. In this case, the operator lays the joystick 62k onto the induction direction side while pressing the away mode button 61s. Therefore, the capsule endoscope 10 jumps and moves in the induction direction, so that the capsule endoscope 10 can move to a position Pu42 beyond the projection 101 as illustrated by an arrow Yu43. Then the operator releases an operator's finger from the away mode button 61s. As a result, the contact mode is implemented, and the magnetic field generating unit 2 generated the magnetic field having the same condition as that before the away mode button 61s is pressed, so that the capsule endoscope 10 can continuously be guided under the same condition as that before the away mode button 61s is pressed.

The same holds true for the case in which the up mode is selected in addition to the case of the down mode. For example, as illustrated in FIG. 6, in the case that the capsule endoscope 10 is guided along the upper stomach wall 31 in order to observe the interior portion of the lower stomach, when a projection 102 is discovered at a position Pt41, the operator may lay the joystick 62k while pressing the away mode button 61s. Therefore, the capsule endoscope 10 jumps over the projection 102 and moves to a position Pt42 as illustrated by an arrow Yt43. Then the operator releases the operator's finger from the away mode button 61s. Therefore, because the magnetic field having the same condition as that before the away mode button 61s is pressed is generated, the capsule endoscope 10 can continuously be guided under the same condition as that before the away mode button 61s is pressed.

According to the first embodiment, it is not necessary for the operator to think whether the capsule endoscope 10 is located in upper portion or the lower portion of the stomach in consideration of a body position of the subject, and it is not necessary for the operator to set the magnetic field generating condition such that the magnetic attraction force is generated in the direction corresponding to the guidance direction. Therefore, the various behaviors of the capsule endoscope 10 can be implemented only by performing the above simple operation.

In the case that the capsule endoscope 10 is swiveled about the long axis 21a of the capsule endoscope 10 at the position Pu42 beyond the projection 101 in selecting the down mode, the operator lays the joystick 62j onto the front side or the rear side to horizontally tilt the joystick 62j while releasing the operator's finger from the away mode button 61s. The behavior of the operation input unit 60 issues the instruction to the external control unit 4 to tilt and swivel the posture of the capsule endoscope 10 while the instruction of the contact mode is issued, and the magnetic field generating unit 2 generates the magnetic fields corresponding to the instructions. As a result, as illustrated in FIG. 7, while a tailing end of the capsule endoscope 10 is pressed at the position Pu42 against the stomach wall 31 of the bottom, the front end of the capsule endoscope 10 swivels 360° as illustrated by an arrow Y51 after the capsule endoscope 10 is inclined by an angle θ (for example, θ = 45°) with respect to the stomach wall 31.

In this case, the operator can also capture the image in the stomach at an optimum camera angle from the desired position of the stomach wall 31 only by performing the simple operation while the capsule endoscope 10 is not destabilized. The same holds true for the up mode. In the case that the capsule endoscope 10 is swiveled about the long axis 21a at the position Pt42 of the upper stomach wall 31 in selecting the up mode, the operator lays the joystick 62j onto the front side or the rear side to horizontally tilt the joystick 62j while releasing the operator's finger from the away mode button 61s. In this case, while the tailing end of the capsule endoscope 10 is pressed at the position Pt42 against the upper stomach wall 31, the front end of the capsule endoscope 10 swivels 360□ as illustrated by an arrow Y52 after the capsule endoscope 10 is inclined by the angle θ (for example, θ = 45°) with respect to the stomach wall 31.

In the case that the behavior of the capsule endoscope becomes instable by a fluctuation in force applied to the capsule endoscope during the away mode, the operator may release the operator's finger from the away mode button 61s to release the zoom back. As a result, the capsule endoscope 10 returns automatically to the boundary surface and comes to rest. The operator can quickly stabilize the capsule endoscope 10 only by releasing the operator's finger from the away mode button 61s. Even if the behavior of the capsule endoscope 10 becomes instable in the liquid, the operator can continue the in-vivo observation because the capsule endoscope 10 can quickly be immobilized and stabilized in the reference plane. The operator switches between the stable state and the behavior state in the binary manner without setting the condition corresponding to the desired behavior from many complicated magnetic field conditions, so that the capsule endoscope existing in the liquid can stably be induced by the simple operation.

The operator further presses one of the gradient up button 64u and the gradient down button 64d while pressing the away mode button 61s, which allows the adjustment of the gradient of the uniform-gradient magnetic field applied in the away mode. That is, the gradient of the uniform-gradient magnetic field, which is applied in order to move the capsule endoscope 10 away from the boundary surface, can be adjusted while the uniform-gradient magnetic field is applied. The magnitude of the magnetic gradient is proportional to the magnitude of the magnetic attraction force. Therefore, in the away mode, a speed at which the capsule endoscope 10 leaves the boundary surface can be controlled by adjusting the magnitude of the magnetic gradient.

The gradient magnetic field cannot be generated with the completely uniform magnetic gradient in the space in which the capsule endoscope 10 is guided, and the generated magnetic field varies according to the position in the space. Therefore, the magnetic gradient changes in the vertical direction according to the position of the capsule endoscope 10. In this case, the operator can instruct the optimum magnetic gradient using the gradient up button 64u and the gradient down button 64d. Accordingly, the operator continuously presses the away mode button 61s, and the operator presses the gradient up button 64u and the gradient down button 64d to adjust the speed, at which the capsule endoscope 10 leaves the boundary surface, while acknowledging the in-vivo image of the capsule endoscope 10 on the display unit 5, so that the in-vivo image can be observed at the optimum observation speed.

For example, in the away mode, an initial condition of the gradient of the applied uniform-gradient magnetic field is set to a relatively small gradient. The operator presses the gradient up button 64u while acknowledging the in-vivo image of the capsule endoscope 10 on the display unit 5, and the away speed of the capsule endoscope 10 is gradually adjusted, so that the capsule endoscope 10 can be induced at low speed. The image displayed on the display unit 5 changes slowly, the operator can calmly perform the in-vivo observation and the guidance operation, and therefore an observation quality and operability are improved.

The magnetic field control instruction unit 45 stores the gradient of the uniform-gradient magnetic field, which the gradient adjustment instruction unit 64 issues the instruction to apply in the away mode, in the magnetic field gradient storage unit 47. In the away mode, in each time the gradient adjustment instruction unit 64 issues the instruction, the magnetic field control instruction unit 45 causes the magnetic field generating unit 2 to generate the uniform-gradient magnetic field such that the uniform-gradient magnetic field has the gradient in response to the instruction from the gradient adjustment instruction unit 64, and the magnetic field control instruction unit 45 updates the gradient of the uniform-gradient magnetic field stored in the magnetic field gradient storage unit to the latest gradient. In the case that the away mode selector 61 selects the away mode again after the away mode is changed to the contact mode, the magnetic field control instruction unit 45 causes the magnetic field generating unit 2 to generate the uniform-gradient magnetic field corresponding to the away mode such that the magnetic gradient stored in the magnetic field gradient storage unit 47 is provided. In other words, the operation input unit 60 includes the gradient up button 64u and the gradient down button 64d, which are of the gradient instruction units that issue instructions regarding the optimum condition of the magnetic gradient of the generated magnetic field in the case that the away mode is selected. In response to the instruction from the gradient instruction unit, the magnetic field gradient storage unit 47 stores the magnetic gradient of the generated magnetic field as the optimum condition therein in the case that the away mode is selected. In the case that the away mode is selected, the magnetic field control instruction unit 45 causes the magnetic field generating unit 2 to generate the magnetic gradient of the optimum condition stored in the magnetic field gradient storage unit 47.

In the case that the operator presses the gradient up button 64u or the gradient down button 64d to adjust the speed at which the capsule endoscope 10 leaves the boundary surface while pressing the away mode button 61s, and in the case that the operator presses the away mode button 61s to perform the in-vivo observation after releasing the operator's finger from the away mode button 61s to return the capsule endoscope 10 to the boundary surface, the in-vivo observation can be performed while the capsule endoscope 10 is moved away from the boundary surface at the optimum away speed already adjusted. When adjusting once the speed at which the capsule endoscope 10 leaves the boundary surface, the operator can perform the in-vivo observation at the optimum away speed only by pressing the away mode button 61s. Therefore, it is not necessary for the operator to finely adjust the magnetic gradient in the vertical direction with the joystick every time the away mode button 61s is pressed, but the operability is improved.

Additionally, the gradient of the uniform-gradient magnetic field in the away mode is individually stored in each of the up mode and down mode, and the up mode or down mode is returned after switched to another mode. In this case, the external control unit 4 may cause the magnetic field generating unit 2 to generate the uniform-gradient magnetic field in the away mode with the gradient stored in the original up mode or down mode. In this case, the adjustment process of the magnetic gradient can also be simplified.

In the first embodiment, the up mode in which the magnetic induction of the capsule endoscope 10 is performed based on the liquid level that is of the upper boundary surface of the liquid in the stomach or the upper stomach wall and the down mode in which the magnetic induction of the capsule endoscope 10 is performed based on the stomach wall of the bottom that is of the lower boundary surface of the liquid in the stomach are set by way of example. However, the invention is not limited to the first embodiment. For example, a mode in which the magnetic induction of the capsule endoscope 10 is performed based on one of the right and left stomach walls in the stomach may further be set. In the case of this mode, for example, as illustrated in FIG. 6, when the operator discovers a projection 103 at a position Ps41 in the guidance direction while guiding the capsule endoscope 10 along the right stomach wall 31 in order to observe the left interior portion of the stomach, the operator may lay the joystick 62k onto the guidance direction side while pressing the away mode button 61s. As illustrated by an arrow Ys43, the capsule endoscope 10 moves to a position Ps42 beyond the projection 103 by the operation. In the case that the capsule endoscope 10 is swiveled about the long axis 21a at the position Ps42 beyond the projection 103, the operator lays the joystick 62j onto the front side or the rear side to horizontally tilt the joystick 62j while releasing the operator's finger from the away mode button 61s. As a result, as illustrated in FIG. 7, while the tailing end of the capsule endoscope 10 is pressed at the position Ps42 against the right stomach wall 31, the front end of the capsule endoscope 10 swivels 360° as illustrated by an arrow Y53 after the capsule endoscope 10 is inclined by an angle θ (for example, θ = 45°) with respect to the stomach wall 31.

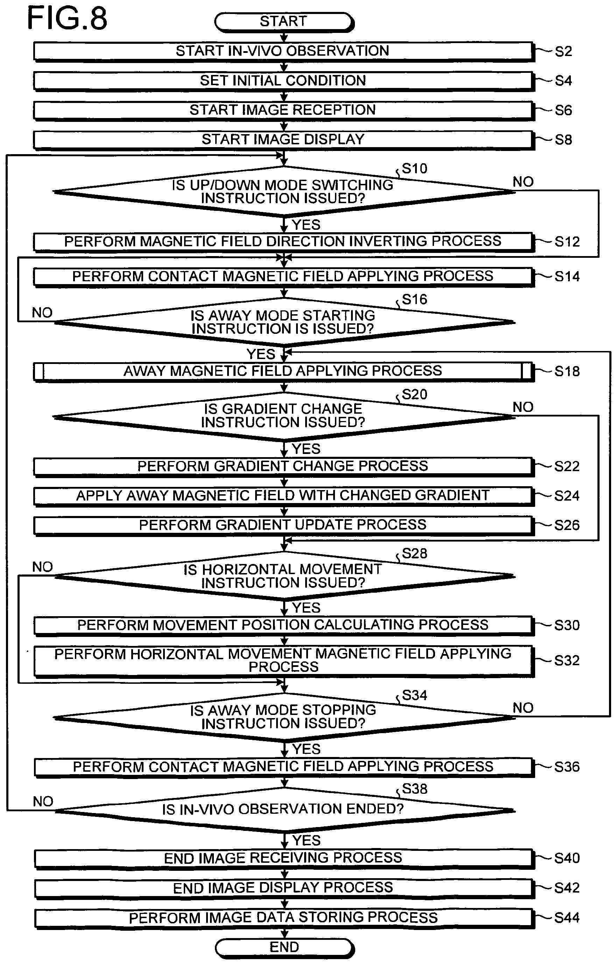

The guidance process of the capsule endoscope 10 of the capsule medical device guidance system 1 will be described below. FIG. 8 is a flowchart illustrating a procedure of the guidance process of the capsule endoscope 10 of the capsule medical device guidance system 1 illustrated in FIG. 1.

As illustrated in FIG. 8, after the instruction information issuing the instruction to start the in-vivo observation is input from the input unit 6 to the external control unit 4 (Step S2), the magnetic field control instruction unit 45 sets the generation of the magnetic field on the initial condition (Step S4), and the magnetic field control instruction unit 45 causes the magnetic field controller 8 to generate the magnetic field on the initial condition. For example, the magnetic field control instruction unit 45 causes the magnetic field generating unit 2 to generate the uniform-gradient magnetic field corresponding to the contact mode in the up mode as the initial condition. The image receiving unit 41 starts image receiving process of sequentially acquiring the in-vivo images received by the transmitting/receiving unit 3 (Step S6), and the image display controller 42 starts image display process of displaying the in-vivo images sequentially received by the transmitting/receiving unit 3 on the display unit 5 (Step S8).

The magnetic field direction switching unit 46 determines whether the magnetic field direction switching instruction unit 63 of the operation input unit 60 issues the instruction to switch between the up and down modes (Step S10). When the magnetic field direction switching unit 46 determines that the magnetic field direction switching instruction unit 63 issues the instruction to switch between the up and down modes (Yes in Step S10), because the boundary surface that becomes the reference plane is vertically inverted, the magnetic field direction switching unit 46 performs magnetic field direction inverting process of issuing the instruction to the magnetic field control instruction unit 45 to invert both the gradient direction of the uniform-gradient magnetic field in the contact mode and the gradient direction of the uniform-gradient magnetic field in the away mode (Step S12). The magnetic field control instruction unit 45 performs contact magnetic field applying process of applying the uniform-gradient magnetic field in the contact mode to the capsule endoscope 10 (Step S14) after inverting the gradient direction of the uniform-gradient magnetic field in the contact mode and the gradient direction of the uniform-gradient magnetic field in the away mode in response to the instruction of the magnetic field direction switching unit 46. As a result, the capsule endoscope 10 is retained so as to be pressed against the reference plane corresponding to the newly-switched up mode or down mode.

On the other hand, when the magnetic field direction switching unit 46 determines that the magnetic field direction switching instruction unit 63 does not issue the instruction to switch between the up and down modes (No in Step S10), because the boundary surface that becomes the reference plane is left without change, the magnetic field control instruction unit 45 performs contact magnetic field applying process of applying the uniform-gradient magnetic field in the contact mode to the capsule endoscope 10 (Step S14) while the gradient direction of the uniform-gradient magnetic field in the contact mode and the gradient direction of the uniform-gradient magnetic field in the away mode are left without change.

The magnetic field control instruction unit 45 determines whether the instruction to start the away mode is issued based on the selection information from the away mode selector 61 (Step S16). When determining that the instruction to start the away mode is not issued (No in Step S16), the magnetic field control instruction unit 45 continues the contact magnetic field applying process in Step S14 to maintain the contact mode. On the other hand, when determining that the instruction to start the away mode is issued (Yes in Step S16), the magnetic field control instruction unit 45 performs an away magnetic field applying process of applying the uniform-gradient magnetic field in the away mode to the capsule endoscope 10 (Step S18). Therefore, the capsule endoscope 10 leaves the reference plane to move in the liquid.

The magnetic field control instruction unit 45 determines whether a gradient change instruction is issued based on the gradient adjustment instruction information from the gradient adjustment instruction unit 64 (Step S20). When determining that the gradient change instruction is issued (Yes in Step S20), the magnetic field control instruction unit 45 changes the gradient of the uniform-gradient magnetic field in the away mode according to the gradient adjustment instruction information (Step S22), and the magnetic field control instruction unit 45 applies the uniform-gradient magnetic field in the away mode with the changed gradient (Step S24). Therefore, the speed at which the capsule endoscope 10 leaves the boundary surface is adjusted. The magnetic field control instruction unit 45 performs gradient update process of updating the gradient in the away mode, which is stored in the magnetic field gradient storage unit 47, to the newly changed gradient (Step S26).

After the gradient update process (Step S26), or when the magnetic field control instruction unit determines that the gradient change instruction is not issued (No in Step S20), the magnetic field control instruction unit 45 determines whether a horizontal movement instruction is issued based on the operation information on the horizontal direction of the horizontal operation input unit 62 (Step S28). When determining that the horizontal movement instruction is issued (Yes in Step S28), the magnetic field control instruction unit 45 calculates the horizontal movement position in response to the instruction of the operation information from the horizontal operation input unit 62 (Step S30), and the magnetic field control instruction unit 45 performs horizontal movement magnetic field applying process to the magnetic field generating unit 2 to apply the magnetic field to the capsule endoscope 10 such that the capsule endoscope 10 moves to the calculated movement position (Step S32). As a result, the capsule endoscope 10 jumps and moves horizontally in the liquid according to the operation process of the operation input unit 60.

Then, the magnetic field control instruction unit 45 determines whether an instruction to stop the away mode is issued based on the selection information of the away mode selector 61 (Step S34). That is, the magnetic field control instruction unit 45 determines whether an instruction to start the contact mode is issued. When determining that the instruction to stop the away mode is issued (Yes in Step S34), the magnetic field control instruction unit 45 performs contact magnetic field applying process of applying the uniform-gradient magnetic field in the contact mode to the capsule endoscope 10 (Step S36), thereby returning the capsule endoscope 10 to the reference plane. On the other hand, when the magnetic field control instruction unit 45 determines that the instruction to stop the away mode is not issued (No in Step S34), the flow returns to Step S18 to continue the away mode.

After the magnetic field control instruction unit 45 performs the contact magnetic field applying process (Step S36) in response to the instruction to stop the away mode, the external control unit 4 determines whether the in-vivo observation is ended based on the instruction information input from the input unit 6 (Step S38). When determining that the in-vivo observation is not ended (No in Step S38), the external control unit 4 returns to Step S10 to continue the in-vivo observation, and the external control unit 4 determines whether the instruction to switch between the up and down mode is issued. When the external control unit 4 determines that the in-vivo observation is ended (Yes in Step S38), the image receiving process of the image receiving unit 41 is ended (Step S40), and the image display process of the image display controller 42 is ended (Step S42). Then the external control unit 4 performs image data storing process of storing the in-vivo image group captured by the capsule endoscope 10 in the storage unit 7 while the in-vivo image group is collected into one folder (Step S44), and the in-vivo observation is ended.

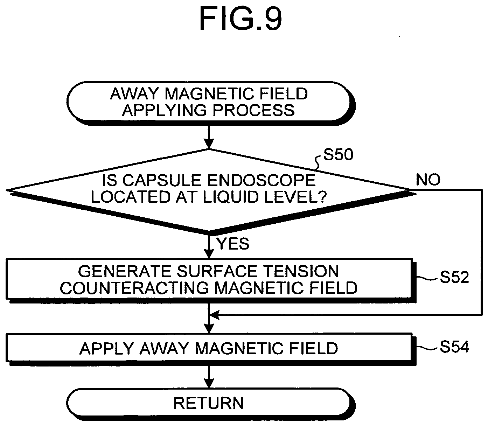

The away magnetic field applying process illustrated in FIG. 8 will be described below. FIG. 9 is a flowchart illustrating a procedure of the away magnetic field applying process illustrated in FIG. 8. As illustrated in FIG. 9, in the away magnetic field applying process, the magnetic field control instruction unit 45 determines whether the capsule endoscope 10 is located in the liquid level (Step S50). When the operator determines that the capsule endoscope 10 is located in the liquid level by acknowledging the imaging screen of the capsule endoscope 10 displayed on the display unit 5, the operator inputs the information indicating that the capsule endoscope 10 is located in the liquid level. The information indicating that the capsule endoscope 10 is located in the liquid level is input from the input unit 6, whereby the magnetic field control instruction unit 45 determines that the capsule endoscope 10 is located in the liquid level.

When determining that the capsule endoscope 10 is located in the liquid level (Yes in Step S50), the magnetic field control instruction unit 45 performs a surface tension counteracting magnetic field generating process to the magnetic field generating unit 2 in order to temporarily generate the magnetic field having high strength that can counteract the surface tension of the liquid level (Step S52). In the case that the capsule endoscope 10 is located in the liquid level, a large magnetic force is required to sink the capsule endoscope 10 in the liquid due to the surface tension. In the case that the operator manually adjusts the gradient of the uniform-gradient magnetic field in the away mode, it is necessary to increase the gradient until the surface tension can be removed. Therefore, once the capsule endoscope 10 sinks under the liquid level, because the capsule endoscope 10 falls at high speed, the image of the display unit 5 changes at high speed, and it is difficult that the operator smoothly performs the in-vivo observation. In the away magnetic field applying process, the surface tension counteracting magnetic field generating process is performed in the case that the capsule endoscope 10 is located in the liquid level, and the capsule endoscope 10 can be guided at the desired speed of the operator while smoothly moved in the liquid with no obstruction of the surface tension.

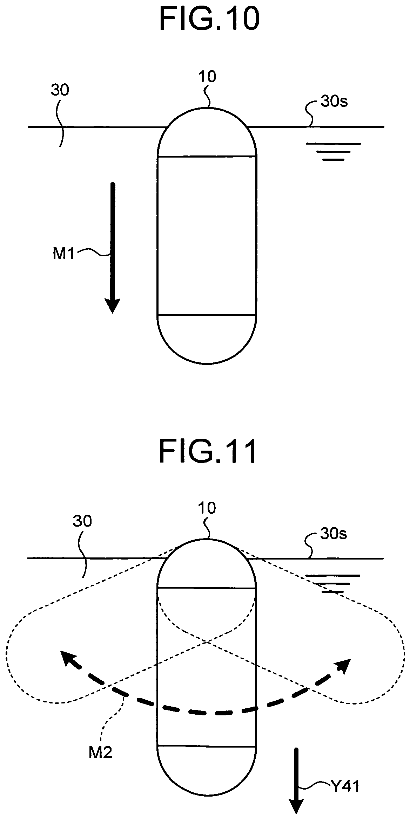

In the surface tension counteracting magnetic field generating process, the magnetic field generating unit 2 temporarily generates the strong magnetic field in the downwardly vertical direction to move the capsule endoscope 10 from a liquid level 30s into the liquid 30 as illustrated by an arrow M1 of FIG. 10. Alternatively, the magnetic field generating unit generates a magnetic field M2 in order that the capsule endoscope 10 performs the tilting behavior at high speed as illustrated in FIG. 11, and the posture of the capsule endoscope 10 may be changed at high speed. In this case, through the tilting behavior, a sidewall of the capsule endoscope 10 exposed from the liquid level 30s wets by the liquid to weaken an influence of the surface tension. Then the magnetic field generating unit 2 generates the magnetic field to downwardly move the capsule endoscope 10 as illustrated by an arrow Y41, whereby the capsule endoscope 10 is moved from the liquid level 30s into the liquid or to the bottom. In this method, the capsule endoscope 10 can be guided from the liquid level 39s into the liquid or to the bottom even by the low magnetic field strength.

When determining that the capsule endoscope 10 is not located in the liquid level (No in Step S50), the magnetic field control instruction unit 45 performs the surface tension counteracting magnetic field generating process (Step S52), the magnetic field control instruction unit 45 causes the magnetic field generating unit 2 to apply the uniform-gradient magnetic field in the away mode to the capsule endoscope 10 (Step S54), and the away magnetic field applying process is ended. It is not always necessary to perform the surface tension counteracting magnetic field generating process, but the surface tension counteracting magnetic field generating process may be performed only in the case that the performance of the surface tension counteracting magnetic field generating process is previously set.

In the capsule medical device guidance system 1 of the first embodiment, the stable guidance of the capsule endoscope existing in the liquid is implemented by performing the pieces of processing of FIGS. 8 and 9.

In the first embodiment, the capsule endoscope 10 drifts in the liquid in which a balance between the buoyant force and the gravity is established by way of example. However, the invention is not limited to the first embodiment.

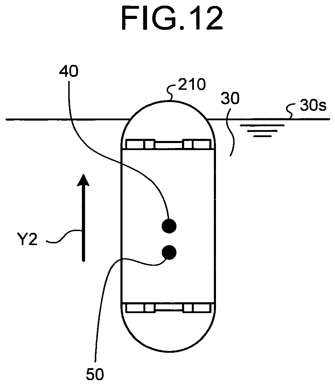

For example, as illustrated in FIG. 12, a capsule endoscope 210 that drifts in the liquid 30 as illustrated by an arrow Y2 may be used. In the capsule endoscope 210, the dispositions of the components of the capsule endoscope 210 are adjusted such that the buoyant force of the capsule endoscope 210 is larger than the gravity of the capsule endoscope 210, whereby a specific weight to the liquid 30 is smaller than 1. The numeral 40 designates a geometric center of the capsule endoscope 210, and the numeral 50 designates the gravity of the capsule endoscope 210.

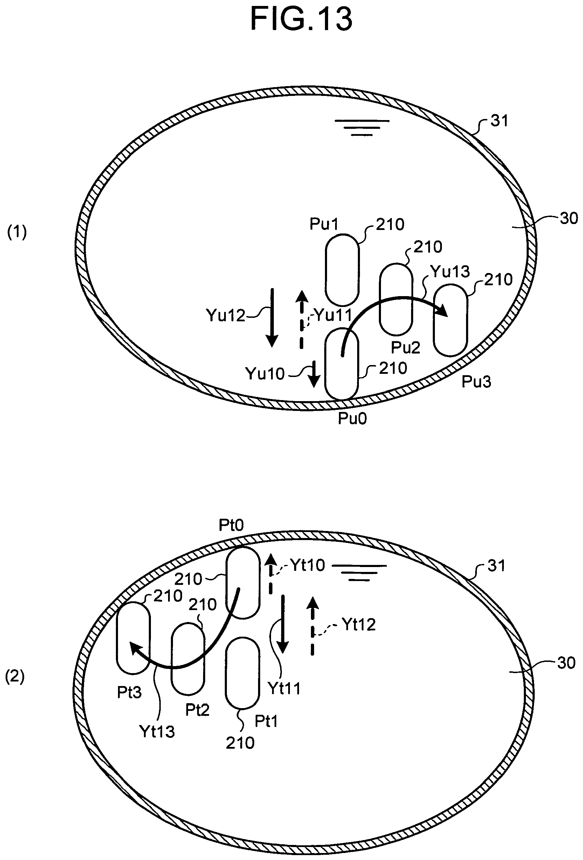

The movement of the capsule endoscope 210 will be described below with reference to FIG 13. The case in which the down mode is selected will be described with reference to FIG. 13(1). As described above, in the case of the down mode, the magnetic induction of the capsule endoscope 210 is performed based on the stomach wall 31 of the bottom in the boundary surface of the liquid. In the case of the down mode, the contact mode corresponds to the mode in which the capsule endoscope 210 is brought into contact with the stomach wall 31 of the bottom. Therefore, in the down mode, the magnetic field direction switching unit 46 issues the instruction to the magnetic field control instruction unit 45 to switch the gradient direction of the uniform-gradient magnetic field in the contact mode to the downward direction of the vertical axis. In the case of the down mode, the away mode corresponds to the mode in which the capsule endoscope 210 is moved upward from the stomach wall 31 of the bottom. In the case that the gradient magnetic field does not act in the vertical direction, the capsule endoscope 210 floats up naturally to the upper stomach wall 31 or the liquid level by the buoyant force thereof. Therefore, in the away mode, because the capsule endoscope 210 may be moved upward from the stomach wall 31 of the bottom by utilizing the floating of the capsule endoscope 210, the magnetic field control instruction unit 45 may stop the generation of the vertically downward gradient magnetic field that is generated in the contact mode by the magnetic field generating unit 2. In order to reduce the floating speed of the capsule endoscope 210, the magnetic field control instruction unit 45 may control the magnetic field generating unit 2 such that the vertically upward magnetic gradient is generated within a range where a total force of the buoyant force and the gravity of the capsule endoscope 210 and the vertically downward magnetic attraction force becomes upward.

Accordingly, in the case that the down mode is selected, in order to retain the capsule endoscope 210 at a position Pu0 of the bottom, the magnetic field control instruction unit 45 causes the magnetic field generating unit 2 to generate the uniform-gradient magnetic field having the vertically downward gradient in which the capsule endoscope 210 is pressed at the position Pu0 of the bottom. As a result, the capsule endoscope 210 retains the stable rest state as illustrated by an arrow Yu10 because the capsule endoscope 210 is pressed at the position Pu0 against the stomach wall 31 of the bottom that becomes the reference.

In the case that the operator presses the away mode button 61s, in order to upwardly move the capsule endoscope 210 away from the position Pu0 of the bottom, the magnetic field control instruction unit 45 stops the uniform-gradient magnetic field having the vertically downward gradient, which is generated by the magnetic field generating unit 2. As a result, by the buoyant force of the capsule endoscope 210, the capsule endoscope 210 floats up from the position Pu0 of the stomach wall 31 and leaves to the position Pu1 as illustrated by an arrow Yu11.

In the case that the operator releases the pressing of the away mode button 61s to select the contact mode, the magnetic field control instruction unit 45 causes the magnetic field generating unit 2 to generate the uniform-gradient magnetic field having the vertically downward gradient, and stabilizes the capsule endoscope 210 by pressing the capsule endoscope 210 against the stomach wall 31 of the bottom at the position Pu0 that becomes the reference as illustrated by an arrow Yu12.

The case in which the tilt operation of the joystick 62k is performed to issue the instruction of the horizontal movement while the operator presses the away mode button 61s will be described. In this case, the magnetic field control instruction unit 45 causes the magnetic field generating unit 2 to generate the uniform-gradient magnetic field having the magnetic gradient in the direction corresponding to the direction of the instruction of the horizontal movement while causing the magnetic field generating unit 2 to halt to generate the uniform-gradient magnetic field having the vertically downward gradient. As a result, the capsule endoscope 210 moves horizontally in the liquid from the position Pu0 to the position Pu3 through the position Pu2 while jumping obliquely upward as illustrated by an arrow Yu13.

The case in which the operator operates the up/down mode changeover switch 63s to switch from the down mode to the up mode will be described with reference to FIG. 13(2). In the case of the up mode, the magnetic induction of the capsule endoscope 210 is performed based on the upper stomach wall 31 or the liquid level in the boundary surface of the liquid 30. In the case of the up mode, the contact mode corresponds to the mode in which the capsule endoscope 210 is retained while being in contact with the upper stomach wall 31 or the liquid level. Therefore, in the contact mode of the up mode, because the capsule endoscope 210 may come into contact with the upper stomach wall 31 or the liquid level by utilizing the floating of the capsule endoscope 210, the magnetic field control instruction unit 45 may control the magnetic field generating unit 2 such that the magnetic field generating unit 2 does not generates at least the uniform-gradient magnetic field having the vertically downward gradient. In the case of the up mode, because the away mode corresponds to the mode in which the capsule endoscope 210 is moved downward from the upper stomach wall 31 or the liquid level, similarly to the capsule endoscope 10, the magnetic field control instruction unit 45 causes the magnetic field generating unit 2 to generate the magnetic field having the magnetic attraction force in the downward direction of the vertical axis. The magnetic field direction switching unit 46 issues the instruction to the magnetic field control instruction unit 45 to switch the gradient direction of the uniform-gradient magnetic field in the away mode of the up mode to the downward direction of the vertical axis.

Accordingly, in the case that the up mode is selected, in order to retain the capsule endoscope 210 at the position Pt0 of the upper stomach wall 31, the magnetic field control instruction unit 45 controls the magnetic field generating unit 2 such that the magnetic field generating unit 2 does not generate at least the uniform-gradient magnetic field having the vertically downward gradient. As a result, the capsule endoscope 210 retains the stable rest state as illustrated by the arrow Yt10, because the capsule endoscope 210 floats up and comes into contact with the position Pt0 of the upper stomach wall 31 that becomes the reference by the buoyant force thereof.

In the case that the operator presses the away mode button 61s, in order to downwardly move the capsule endoscope 210 away from the position Pt0 of the upper stomach wall 31, the magnetic field control instruction unit 45 causes the magnetic field generating unit 2 to generate the uniform-gradient magnetic field having the vertically downward gradient in which the capsule endoscope 210 is moved downward. As a result, the capsule endoscope 210 sinks from the position Pt0 of the stomach wall 31 into the liquid 30 and leaves to a position Pt1 as illustrated by the arrow Yt11.

In the case that the operator releases the pressing of the away mode button 61s, in order to correspond to the case in which the contact mode is selected, the magnetic field control instruction unit 45 causes the magnetic field generating unit 2 to stop the generation of the uniform-gradient magnetic field having the vertically downward gradient. As a result, the capsule endoscope 210 retains the stable rest state as illustrated by the arrow Yt12, because the capsule endoscope 210 floats up and comes into contact with the position Pt0 of the upper stomach wall 31 that becomes the reference by the buoyant force thereof.

The case in which the tilt operation of the joystick 62k is performed to issue the instruction of the horizontal movement while the operator presses the away mode button 61s will be described. In this case, the magnetic field control instruction unit 45 causes the magnetic field generating unit 2 to generate the uniform-gradient magnetic field having the magnetic gradient in the direction corresponding to the direction of the instruction of the horizontal movement while causing the magnetic field generating unit 2 to generate the uniform-gradient magnetic field having the vertically downward gradient corresponding to the away mode. As a result, the capsule endoscope 210 moves horizontally in the liquid from the position Pt0 to the position Pt3 through the position Pt2 while jumping obliquely downward as illustrated by the arrow Yt13. Alternatively, in the contact mode of the up mode, the magnetic field control instruction unit 45 causes the magnetic field generating unit 2 to generate the uniform-gradient magnetic field having the gradient in the upward direction of the vertical axis, and the capsule endoscope 210 may be pressed against the upper stomach wall 31 or the liquid level to securely bring the capsule endoscope 210 into contact with the stomach wall 31.

Thus, in the capsule medical device guidance system 1, the capsule endoscope 210 that floats in the liquid can stably be guided by controlling the generation of the uniform-gradient magnetic field having the gradient in the vertical axis direction in the contact mode and the away mode of the up mode and the down mode.

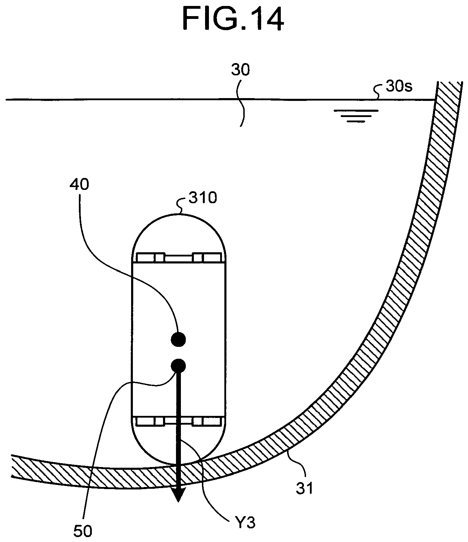

A capsule endoscope 310 that sinks in the liquid as illustrated by an arrow Y3 of FIG. 14 can also be used. In the capsule endoscope 310, the dispositions of the components of the capsule endoscope 310 are adjusted such that the gravity of the capsule endoscope 310 is larger than the buoyant force of the capsule endoscope 310, whereby the specific weight to the liquid 30 is larger than 1.

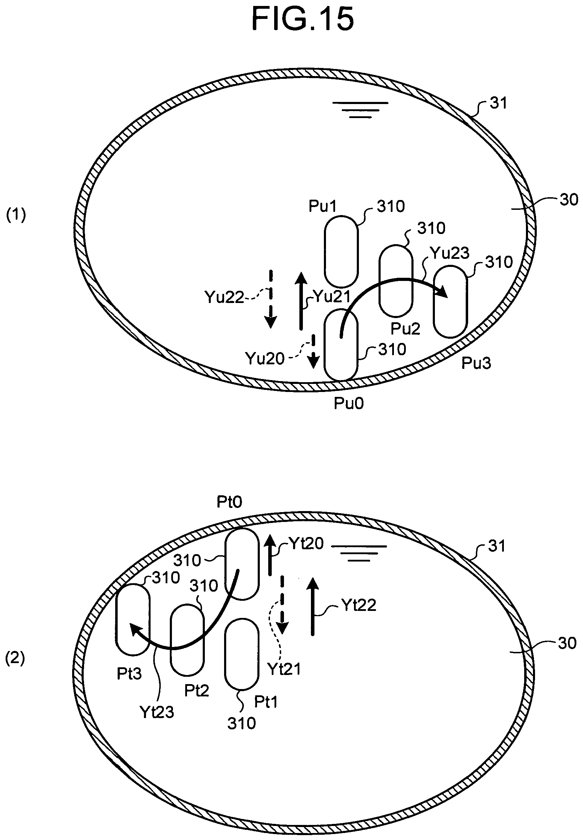

The movement of the capsule endoscope 310 will be described below with reference to FIG. 15. The case in which the down mode is selected will be described with reference to FIG. 15(1). In the case of the down mode, the magnetic induction of the capsule endoscope 310 is performed based on a stomach wall 31 of the bottom in the boundary surface of the liquid. In the case of the down mode, the contact mode corresponds to the mode in which the capsule endoscope 310 is brought into contact with the stomach wall 31 of the bottom. In the case that the gradient magnetic field does not act in the vertical direction, the capsule endoscope 310 sinks naturally to the stomach wall 31 of the bottom by the gravity thereof. Therefore, in the contact mode of the down mode, because the capsule endoscope 310 may come into contact with the stomach wall 31 of the bottom by utilizing the sinking of the capsule endoscope 310, the magnetic field control instruction unit 45 may control the magnetic field generating unit 2 such that the magnetic field generating unit 2 does not generates at least the vertically upward gradient magnetic field. In the case of the down mode, because the away mode corresponds to the mode in which the capsule endoscope 310 is moved upward from the upper stomach wall 31 of the bottom, similarly to the capsule endoscope 310, the magnetic field control instruction unit 45 causes the magnetic field generating unit 2 to generate the magnetic field having the magnetic attraction force in the upward direction of the vertical axis. The magnetic field direction switching unit 46 issues the instruction to the magnetic field control instruction unit 45 to switch the gradient direction of the uniform-gradient magnetic field in the away mode from the down mode to the upward direction of the vertical axis.

Accordingly, in the case that the up mode is selected, in order to retain the capsule endoscope 310 at the position Pu0 of the bottom, the magnetic field control instruction unit 45 controls the magnetic field generating unit 2 such that the magnetic field generating unit 2 does not generate at least the uniform-gradient magnetic field having the vertically upward gradient. As a result, the capsule endoscope 310 retains the stable rest state as illustrated by an arrow Yu20, because the capsule endoscope 310 sinks and comes into contact with the position Pu0 of the bottom that becomes the reference by the gravity thereof.

In the case that the operator presses the away mode button 61s, in order to upwardly move the capsule endoscope 310 away from the position Pu0 of the bottom, the magnetic field control instruction unit 45 causes the magnetic field generating unit 2 to generate the uniform-gradient magnetic field having the vertically upward gradient in which the capsule endoscope 310 is moved upward. As a result, the capsule endoscope 310 floats up from the position Pu0 of the stomach wall 31 and leaves to a position Pu1 as illustrated by an arrow Yu21.

In the case that the operator releases the pressing of the away mode button 61s to select the contact mode, the magnetic field control instruction unit 45 causes the magnetic field generating unit 2 to stop the generation of the uniform-gradient magnetic field having the vertically upward gradient. As a result, the capsule endoscope 310 retains the stable rest state as illustrated by an arrow Yu22, because the capsule endoscope 310 sinks and comes into contact with the position Pu0 of the stomach wall 31 of the bottom that becomes the reference by the gravity thereof.

The case in which the tilt operation of the joystick 62k is performed to issue the instruction of the horizontal movement while the operator presses the away mode button 61s will be described. In this case, the magnetic field control instruction unit 45 causes the magnetic field generating unit 2 to generate the uniform-gradient magnetic field having the magnetic gradient in the direction corresponding to the direction of the instruction of the horizontal movement while causing the magnetic field generating unit 2 to generate the uniform-gradient magnetic field having the vertically upward gradient corresponding to the away mode. As a result, the capsule endoscope 310 moves horizontally in the liquid from the position Pu0 to the position Pu3 through the position Pu2 while jumping obliquely upward as illustrated by an arrow Yu23. Alternatively, in the contact mode of the down mode, the magnetic field control instruction unit 45 causes the magnetic field generating unit 2 to generate the uniform-gradient magnetic field having the gradient in the upward direction of the vertical axis, and the capsule endoscope 310 may be pressed against the upper stomach wall 31 of the bottom to securely bring the capsule endoscope 310 into contact with the stomach wall 31.

The case in which the operator operates the up/down mode changeover switch 63s to switch from the down mode to the up mode will be described with reference to FIG. 15(2). In the case of the up mode, the magnetic induction of the capsule endoscope 310 is performed based on the upper stomach wall 31 or the liquid level in the boundary surface of the liquid 30. In the case of the up mode, because the contact mode corresponds to the mode in which the capsule endoscope 310 is retained while brought into contact with the upper stomach wall 31 or the liquid level, similarly to the capsule endoscope 10, the magnetic field direction switching unit 46 issues the instruction to the magnetic field control instruction unit 45 to switch the gradient of the uniform-gradient magnetic field to the upward direction of the vertical axis. In the case of the up mode, the away mode corresponds to the mode in which the capsule endoscope 310 is moved downward from the upper stomach wall 31 or the liquid level. In the away mode of the up mode, because the capsule endoscope 310 may sink into the liquid by utilizing the sinking of the capsule endoscope 310, the magnetic field control instruction unit 45 may control the magnetic field generating unit 2 such that the magnetic field generating unit 2 does not generates at least the vertically upward gradient magnetic field. In order to reduce the floating speed of the capsule endoscope 310, the magnetic field control instruction unit 45 may control the magnetic field generating unit 2 such that the vertically upward magnetic gradient is generated within a range where a total force of the buoyant force and the gravity of the capsule endoscope 310 and the vertically upward magnetic attraction force becomes downward.

Accordingly, in the case that the up mode is selected, in order to retain the capsule endoscope 310 at the position Pt0 of the upper stomach wall 31, the magnetic field control instruction unit 45 causes the magnetic field generating unit 2 to generate the uniform-gradient magnetic field having the vertically upward gradient. As a result, the capsule endoscope 310 retains the stable rest state as illustrated by an arrow Yt20 because the capsule endoscope 310 is pressed at the position Pt0 against the upper stomach wall 31 that becomes the reference.

In the case that the operator presses the away mode button 61s, the magnetic field control instruction unit 45 controls the magnetic field generating unit 2 such that the magnetic field generating unit 2 does not generate at least the uniform-gradient magnetic field having the vertically upward gradient in which the capsule endoscope 310 is moved downward from the position Pt0 of the upper stomach wall 31. As a result, the capsule endoscope 310 sinks in the liquid 30 from the position Pt0 of the stomach wall 31 and leaves to the position Pt1 as illustrated by an arrow Yt21 by the gravity thereof.

In the case that the operator releases the pressing of the away mode button 61s, in order to correspond to the case in which the contact mode is selected, the magnetic field control instruction unit 45 causes the magnetic field generating unit 2 to generate the uniform-gradient magnetic field having the vertically upward gradient, and stabilizes the capsule endoscope 310 by pressing the capsule endoscope 310 against the upper stomach wall 31 at the position Pt0 that becomes the reference as illustrated by an arrow Yt22.

The case in which the tilt operation of the joystick 62k is performed to issue the instruction of the horizontal movement while the operator presses the away mode button 61s will be described. In this case, the magnetic field control instruction unit 45 causes the magnetic field generating unit 2 to generate the uniform-gradient magnetic field having the magnetic gradient in the direction corresponding to the direction of the instruction of the horizontal movement while causing the magnetic field generating unit 2 to halt to generate the uniform-gradient magnetic field having the vertically upward gradient. As a result, the capsule endoscope 310 moves horizontally in the liquid from the position Pt0 to the position Pt3 through the position Pt2 while jumping obliquely downward as illustrated by an arrow Yt23.

Thus, in the capsule medical device guidance system 1, the capsule endoscope 310 that floats in the liquid can stably be guided by controlling the generation of the uniform-gradient magnetic field having the gradient in the vertical axis direction in the contact mode and the away mode of the up mode and the down mode.

As described above, in the capsule medical device guidance system 1, the contact mode in which the capsule endoscope 10 is brought into contact with the desired boundary surface in the plural boundary surfaces of the liquid 30 in the subject and the away mode in which the capsule endoscope 10 is moved away from the desired boundary surface are set, the external control unit 4 controls the magnetic field generating unit 2 such that the total force in the liquid of the buoyant force of the capsule endoscope 10, 210, or 310, the gravity of the buoyant force of the capsule endoscope 10, 210, or 310, and the magnetic attraction force is oriented toward the desired boundary surface side in the case that the contact mode is selected, and the external control unit 4 controls the magnetic field generating unit 2 such that the total force in the liquid of the buoyant force of the capsule endoscope 10, 210, or 310, the gravity of the buoyant force of the capsule endoscope 10, 210, or 310, and the magnetic attraction force is oriented toward the direction except the direction oriented toward the desired boundary surface side in the case that the away mode is selected, thereby implementing the stable guidance of the capsule endoscope 10, 210, or 310 existing in the liquid.

### (Second Embodiment)

A second embodiment will be described below. In the description of the second embodiment, a function of detecting the behavior of the capsule endoscope 10 is added to automatically set the away speed of the capsule endoscope 10 to a desired speed based on the away behavior of the capsule endoscope 10 from the boundary surface.

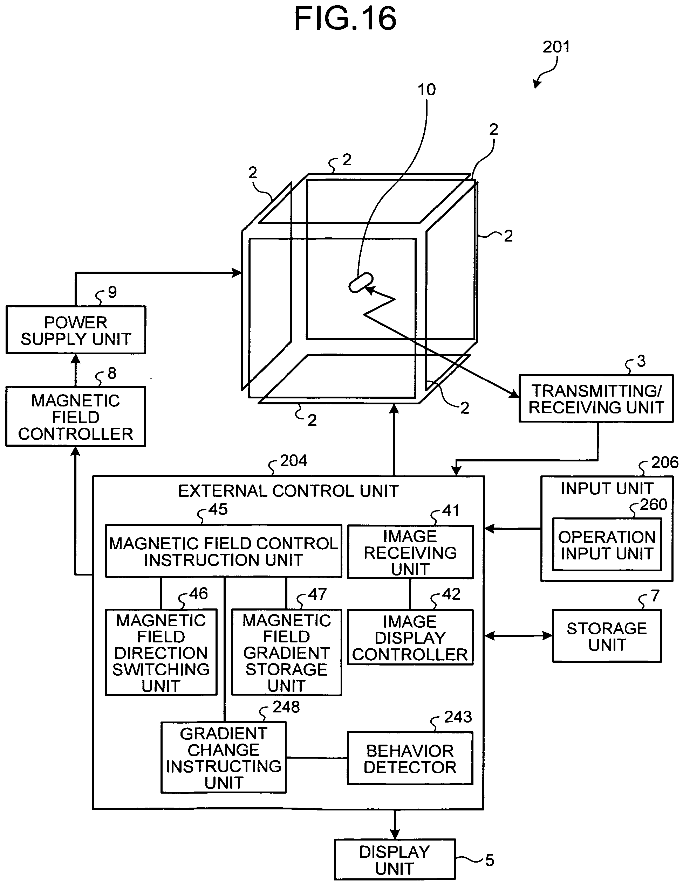

FIG. 16 is a schematic diagram illustrating an entire configuration of a capsule medical device guidance system of the second embodiment. As illustrated in FIG. 16, a capsule medical device guidance system 201 of the second embodiment includes an external control unit 204 instead of the external control unit 4 of FIG. 1, and includes an input unit 206 instead of the input unit 6 of FIG. 1. Compared with the external control unit 4 of FIG. 1, the external control unit 204 further includes a behavior detector 243 and a gradient change instructing unit 248.

The behavior detector 243 detects the behavior of the capsule endoscope 10. The behavior detector 243 detects the behavior speed of the capsule endoscope 10 in the body. The behavior detector 243 detects the behavior speed of the capsule endoscope 10 in the body based on data transmitted from the capsule endoscope 10 to the transmitting/receiving unit 3. For example, the behavior detector 243 continuously detects the received electric field intensity of the signal transmitted from the capsule endoscope 10, and detects the behavior speed of the capsule endoscope 10 from the change in received electric field intensity of the signal. The behavior detector 243 analyzes the image captured by the capsule endoscope 10, and detects the behavior speed of the capsule endoscope 10.

The gradient change instructing unit 248 issues an instruction to the magnetic field control instruction unit 45 to change the gradient of the vertical uniform-gradient magnetic field, which is generated in the away mode by the magnetic field generating unit 2, in a stepwise manner. Based on the behavior detection result of the capsule endoscope 10 by the behavior detector 243, the gradient change instructing unit 248 detects at which gradient of the uniform-gradient the capsule endoscope 10 starts the behavior to leave the boundary surface that becomes the reference in the away mode. The gradient change instructing unit 248 notifies the magnetic field control instructing unit 45 of the gradient at which the behavior to leave the boundary surface that becomes the reference in the away mode is started as the optimum gradient of the uniform-gradient magnetic field in the away mode. The magnetic field control instruction unit 45 sets the gradient notified as the optimum gradient to the optimum gradient of the magnetic gradient of the uniform-gradient magnetic field in the away mode, and caused the magnetic field generating unit 2 to generate the uniform-gradient magnetic field having the gradient of the set optimum gradient.

In the case that an operation input unit 260 issues the instruction to start the away mode, the external control unit 204 determines at which gradient the capsule endoscope 10 starts to leave the boundary surface while increasing the gradient of the uniform-gradient magnetic field in the away mode from the initial condition in the stepwise manner, and the external control unit 204 sets the gradient at which the capsule endoscope 10 starts to leave the boundary surface to the optimum gradient of the uniform-gradient magnetic field in the away mode. Accordingly, in the case that the away mode is set, the external control unit 204 automatically sets the gradient of the uniform-gradient magnetic field, at which the capsule endoscope 10 starts to leave the boundary surface. As a result, it is not necessary that the operator operate the gradient up button 64u and the gradient down button 64d to adjust the gradient of the uniform-gradient magnetic field while observing the image, but the capsule endoscope 10 can always be guided at the optimum induction speed.

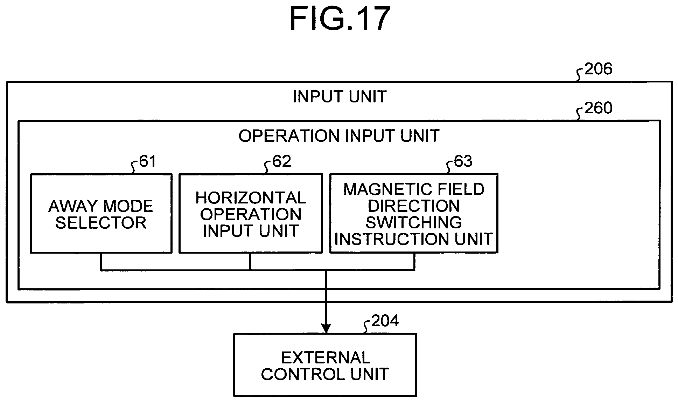

In the case that the away mode is set, the external control unit 204 automatically sets the gradient of the uniform-gradient magnetic field, at which the capsule endoscope 10 starts to leave the boundary surface. Therefore, as illustrated in FIG. 17, the gradient adjustment instruction unit 64 is removed in the operation input unit 260 compared with the operation input unit 60 of FIG. 3.

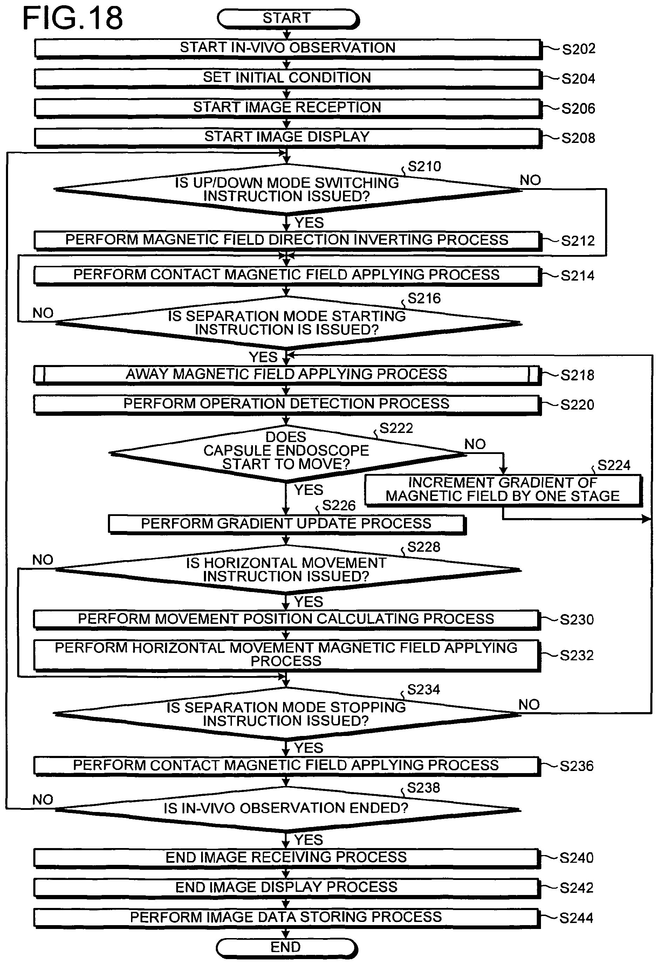

The guidance process of the capsule endoscope 10 of the capsule medical device guidance system 201 illustrated in FIG. 16 will be described below with reference to FIG. 18. FIG. 18 is a flowchart illustrating a procedure of the guidance process of the capsule endoscope 10 of the capsule medical device guidance system 201 illustrated in FIG. 16.

As illustrated in FIG. 18, similarly to Steps S2 to S8 of FIG. 8, after the instruction to start the in-vivo observation is issued (Step S202), the magnetic field control instruction unit 45 sets the generation of the magnetic field on the initial condition (Step S204), the magnetic field control instruction unit 45 causes the magnetic field controller 8 to generate the magnetic field on the initial condition, the image receiving unit 41 starts the image receiving process (Step S206), and the image display controller 42 starts the image display process (Step S208).

Similarly to Step S10 of FIG. 8, the magnetic field direction switching unit 46 determines whether the instruction to switch between the up and down modes is issued (Step S210). When determining that the instruction to switch between the up and down modes is issued (Yes in Step S210), the magnetic field direction switching unit 46 performs magnetic field direction inverting process (Step S212) similarly to Step S12 of FIG. 8, and the magnetic field control instruction unit 45 inverts both the gradient direction of the uniform-gradient magnetic field in the contact mode and the gradient direction of the uniform-gradient magnetic field in the away mode. When the magnetic field direction switching unit 46 determines that the instruction to switch between the up and down modes is not issued (No in Step S210), or after the magnetic field direction inverting process is ended (Step S212), the magnetic field direction switching unit 46 performs the contact magnetic field applying process (Step S214) similarly to Step S14 of FIG. 8. Similarly to Step S16 of FIG. 8, the magnetic field control instruction unit 45 determines whether the instruction to start the away mode is issued (Step S216). When determining that the instruction to start the away mode is not issued (No in Step S216), the magnetic field control instruction unit 45 continues the contact magnetic field applying process in Step S214 to maintain the contact mode. On the other hand, when determining that the instruction to start the away mode is issued (Yes in Step S216), the magnetic field control instruction unit 45 performs the away magnetic field applying process of applying the uniform-gradient magnetic field in the away mode to the capsule endoscope 10 (Step S218). In this case in response to the instruction of the gradient change instructing unit 248, the magnetic field control instruction unit 45 causes the magnetic field generating unit 2 to generate the uniform-gradient magnetic field in the away mode at the gradient of the initial condition.

The behavior detector 243 performs operation detection process of detecting the behavior speed of the capsule endoscope 10 in the body (Step S220), and outputs the detected behavior speed to the gradient change instructing unit 248. In this case, in the away mode, the behavior detector 243 detects the behavior speed at which the capsule endoscope 10 leaves vertically the boundary surface that becomes the reference. The gradient change instructing unit 248 determines whether the capsule endoscope 10 starts to leave the boundary surface (Step S222) based on the detection result of the behavior detector 243.

When determining that the capsule endoscope 10 does not start to leave the boundary surface (No in Step S222), the gradient change instructing unit 248 determines that the magnetic attraction force acting on the capsule endoscope 10 lacks, and the gradient change instructing unit 248 issues the instruction to the magnetic field control instruction unit 45 to increase the gradient of the uniform-gradient magnetic field in the away mode by one step in order to enhance the magnetic attraction force acting on the capsule endoscope 10 (Step S224). The magnetic field control instruction unit 45 increases the gradient of the uniform-gradient magnetic field in the away mode by one step to perform the away magnetic field applying process (Step S218). The behavior detector 243 performs the operation detection process (Step S220), and the gradient change instructing unit 248 determines whether the capsule endoscope 10 starts to leave the boundary surface again (Step S222). The pieces of processing in Steps S218 to S224 are repeated until the gradient change instructing unit 248 determines that the capsule endoscope 10 starts to leave the boundary surface.

When determining that the capsule endoscope 10 starts to leave the boundary surface (Yes in Step S222), the gradient change instructing unit 248 performs the gradient update process updating the optimum condition of the gradient in the away mode stored in the magnetic field gradient storage unit 47 by setting the gradient of the uniform-gradient magnetic field, which is generated in the last away magnetic field applying process by the magnetic field generating unit 2, as the gradient of the uniform-gradient magnetic field in the away mode (Step S226).

Similarly to Steps S28 to S32 of FIG. 8, the magnetic field control instruction unit 45 performs the horizontal movement instruction determination process (Step S228), horizontal movement position calculating process (Step S230), and horizontal movement magnetic field applying process (Step S232). Similarly to Step S34 of FIG. 8, the magnetic field control instruction unit 45 performs away mode stopping instruction determination process (Step S234). When determining that the instruction to stop the away mode is issued (Yes in Step S234), the magnetic field control instruction unit 45 performs the contact magnetic field applying process of applying the uniform-gradient magnetic field in the contact mode to the capsule endoscope 10 (Step S236), thereby returning the capsule endoscope 10 to the reference plane. On the other hand, when the magnetic field control instruction unit 45 determines that the instruction to stop the away mode is not issued (No in Step S234), the flow returns to Step S218 to continue the away mode.

Similarly to Step S38 of FIG. 8, the external control unit 204 determines whether the in-vivo observation is ended based on the instruction information input from the input unit 206 (Step S238). When determining that the in-vivo observation is not ended (No in Step S238), the external control unit 204 returns to Step S210 to continue the in-vivo observation, and the external control unit 204 determines whether the instruction to switch between the up and down mode is issued. When the external control unit 204 determines that the in-vivo observation is ended (Yes in Step S238), the image receiving process of the image receiving unit 41 is ended (Step S240), and the image display process of the image display controller 42 is ended (Step S242). Then the external control unit 204 performs the image data storing process (Step S244), and the in-vivo observation is ended.

In the capsule medical device guidance system 201 of the second embodiment, the gradient of the uniform-gradient magnetic field applied to the capsule endoscope 10 in the away mode is automatically adjusted by performing the pieces of processing of FIG. 18, so that a burden of the gradient adjustment on the operator can be reduced.

In the second embodiment, the capsule endoscope 10 includes an acceleration sensor, and the behavior detector 243 may detect the behavior speed of the capsule endoscope 10 based on the acceleration information on the acceleration sensor of the capsule endoscope 10.

In Step S222, the gradient change instructing unit 248 does not determine whether the capsule endoscope 10 starts to leave the boundary surface, but the gradient change instructing unit 248 may determine whether the away speed becomes a predetermined behavior speed. That is, the gradient of the uniform-gradient magnetic field in the away mode may be adjusted such that the capsule endoscope 10 is moved away from the boundary surface at a previously-set desired speed. In this case, because the speed at which the capsule endoscope 10 leaves the boundary surface is automatically set to the desired away speed previously set by the operator, the operator can guide the capsule endoscope 10 at the optimum speed, and therefore the operator can further facilitate the in-vivo observation.

In the capsule medical device guidance system of the second embodiment, a magnetic field generating unit or magnetic field reflecting unit is provided in the capsule endoscope 10, plural magnetic field sensors are provided so as to surround the capsule endoscope 10 similarly to the magnetic field generating unit 2, and the position and the posture of the capsule endoscope 10 may be detected based on detection results of the magnetic field sensors. Modification of Second Embodiment

### (Modification of second embodiment)

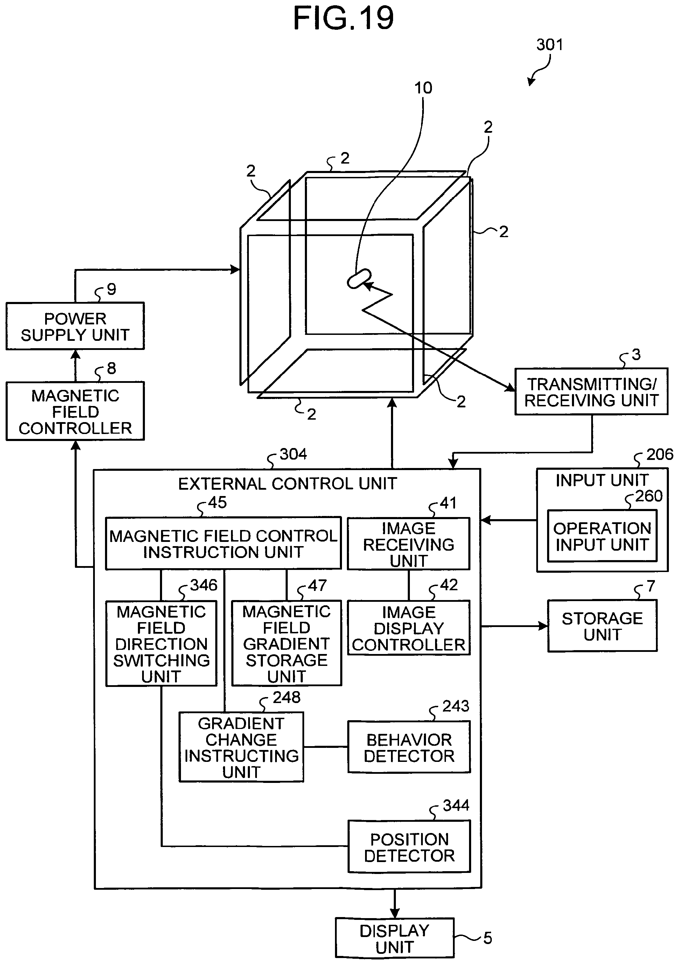

A modification of the second embodiment will be described below. FIG. 19 is a schematic diagram illustrating an entire configuration of a capsule medical device guidance system according to the modification of the second embodiment. As illustrated in FIG. 19, a capsule medical device guidance system 301 of the modification of the second embodiment includes an external control unit 304 instead of the external control unit 204 of FIG. 16. Compared with the external control unit 204 of FIG. 16, the external control unit 304 further includes a position detector 344. The external control unit 304 includes a magnetic field direction switching unit 346 instead of the magnetic field direction switching unit 46 of FIG. 16.

The position detector 344 detects whether the capsule endoscope 10 is located on the upper side or the lower side in the internal organ. For example, the position detector 344 detects whether the capsule endoscope 10 is located on the upper side or the lower side in the internal organ from the image, which is captured by the capsule endoscope and received by the image receiving unit 41, based on whether an image pattern unique to the liquid level or the inner wall of the internal organ is exists in the image.

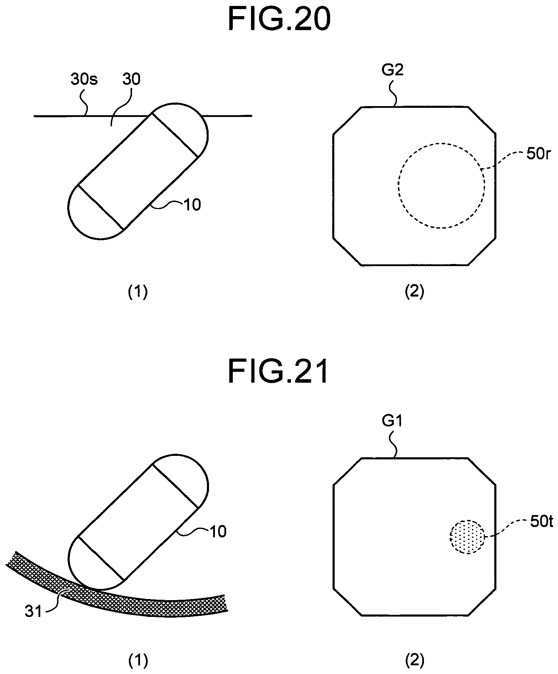

The case in which the capsule endoscope 10 is located at the liquid level will be described with reference to FIG. 20. As illustrated in FIG. 20(1), in the case that the capsule endoscope 10 is located at the liquid level, the front end of the capsule endoscope 10 is exposed from the liquid level 30s. The imaging visual field of one of the imaging units 11A and 11B is spread from the front end of the capsule endoscope 10. Therefore, in the case that the front end of the capsule endoscope 10 is exposed from the liquid level 30s as illustrated in FIG. 20(1), a boundary 50r with the liquid level 30s is displayed into a ring shape like an image G2 of FIG. 20(2) by a climb of the liquid 30 to the side surface of the capsule endoscope 10 due to the surface tension and the reflection of the illumination light from the illumination units 13A and 13B. The position detector 344 determines whether the ring image pattern exists in the image captured by the capsule endoscope 10. The determination that the capsule endoscope 10 exists at the liquid level, namely, in the upper side of the stomach is determined, when the ring image pattern exists in the image.

The case in which the capsule endoscope 10 is in contact with the stomach wall 31 will be described with reference to FIG. 21. As illustrated in FIG. 21(1), in the case that the capsule endoscope 10 is in contact with the stomach wall 31, the front end of the capsule endoscope 10 is pressed against the stomach wall 31. Therefore, in the case of FIG. 21(1), a contact portion 50t between the stomach wall 31 and the front end of the capsule endoscope 10 is circularly displayed like an image G1 of FIG. 21(2). Accordingly, the position detector 344 determines whether the circular image pattern exists in the image captured by the capsule endoscope 10. The determination that the capsule endoscope 10 is in contact with the stomach wall 31 is made when the circular image pattern exists in the image. When determining that the unique image pattern to the stomach wall 31 exists, the position detector 344 determines which one of the imaging units 11A and 11B captures the image, and the position detector 344 determines which vertical direction the imaging unit that captures the image is oriented toward from the posture of the capsule endoscope 10 in capturing the image. Therefore, the position detector 344 determines whether the capsule endoscope 10 is in contact with the upper stomach wall 31 or the bottom stomach wall 31. When determining that the ring image pattern and the circular image pattern do not exist in the image captured by the capsule endoscope 10, the position detector 344 determines that the capsule endoscope 10 drifts in the liquid.

The magnetic field direction switching unit 346 determines whether the state in which the capsule endoscope drifts in the liquid correspond to the up mode or the down mode based on the position of the capsule endoscope 10 detected in the internal organ by the position detector 344, and the magnetic field direction switching unit 346 switches the gradient direction of the uniform-gradient magnetic field, which is generated in each of the contact mode and the away mode according to the determined up mode or down mode, to a predetermined direction. When the position detector 344 determines that the capsule endoscope 10 drifts in the liquid, the magnetic field direction switching unit 346 switches the gradient direction of the uniform-gradient magnetic field in the contact mode and the away mode according to a default mode in the up mode and the down mode.

That is, in the capsule medical device guidance system 301, the determination whether the capsule endoscope 10 is located in the position corresponding to the up mode or the down mode is detected, the determination whether the up mode or the down mode corresponding to the position of the capsule endoscope 10 is made, and the gradient direction of the uniform-gradient magnetic field generated in each of the contact mode and the away mode is automatically set. Therefore, the gradient direction of the uniform-gradient magnetic field in each of the contact mode and the away mode suitable to the position of the capsule endoscope 10 is automatically set, even if the operator cannot set the up mode or the down mode because the operator cannot determine whether the capsule endoscope 10 is in contact with the upper stomach wall or the bottom stomach wall based on the observation of the image.

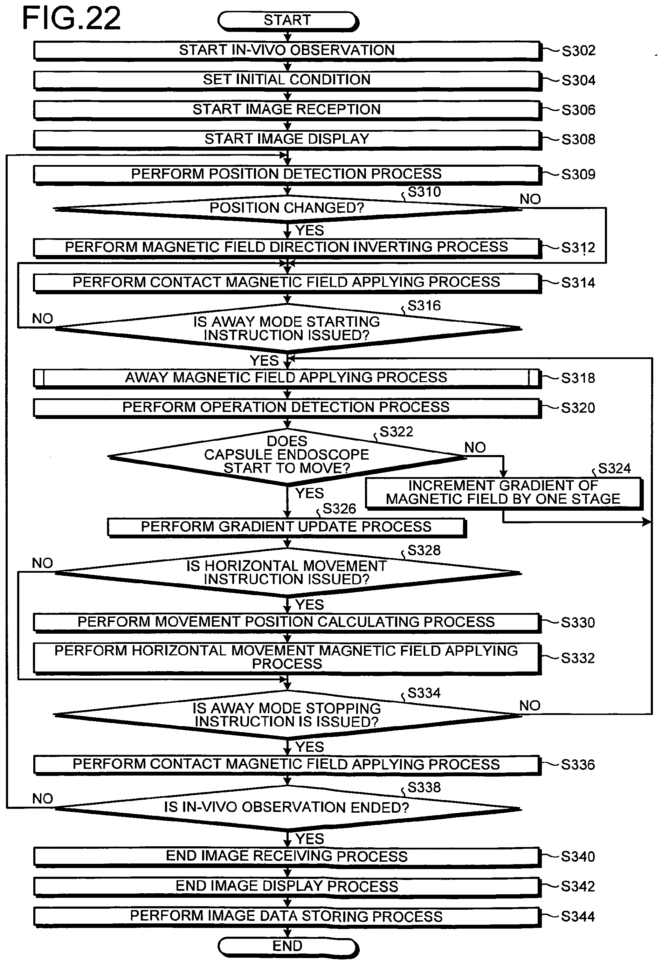

The guidance process of the capsule endoscope 10 of the capsule medical device guidance system 301 illustrated in FIG. 19 will be described below with reference to FIG. 22. FIG. 22 is a flowchart illustrating a procedure of the guidance process of the capsule endoscope 10 of the capsule medical device guidance system 301 illustrated in FIG. 19.

As illustrated in FIG. 22, similarly to Steps S2 to S8 of FIG. 8, after the instruction to start the in-vivo observation is issued (Step S302), the magnetic field control instruction unit 45 sets the generation of the magnetic field on the initial condition (Step S304), the magnetic field control instruction unit 45 causes the magnetic field controller 8 to generate the magnetic field on the initial condition, the image receiving unit 41 starts the image receiving process (Step S306), and the image display controller 42 starts the image display process (Step S308).

The position detector 344 performs the position detection process of determining whether the capsule endoscope 10 is located on the upper side or lower side of the internal organ (Step S309), and the position detector 344 outputs the detection result to the magnetic field direction switching unit 346. The magnetic field direction switching unit 346 determines whether the position of the capsule endoscope 10 detected in the internal organ by the position detector 344 is changed from the upper side to the lower side or from the lower side to the upper side (Step S310).

When determining that the position of the capsule endoscope 10 in the internal organ is changed (Yes in Step S310), the magnetic field direction switching unit 346 determines whether the changed position of the capsule endoscope 10 corresponds to the up mode or the down mode, and performs the magnetic field direction inverting process according to the determined up mode or down mode (Step S312). The magnetic field control instruction unit 45 inverts the gradient directions of the uniform-gradient magnetic fields in the contact mode and the away mode.

On the other hand, when the magnetic field direction switching unit 346 determines that the position of the capsule endoscope 10 in the internal organ is not changed (No in Step S310), or after the magnetic field direction inverting process is ended (Step S312), the magnetic field direction switching unit 346 performs the contact magnetic field applying process (Step S314) similarly to Step S14 of FIG. 8. Similarly to Step S16 of FIG. 8, the magnetic field control instruction unit 45 determines whether the instruction to start the away mode is issued (Step S316). When determining that the instruction to start the away mode is not issued (No in Step S316), the magnetic field control instruction unit 45 continues the contact magnetic field applying process in Step S314 to maintain the contact mode. On the other hand, when determining that the instruction to start the away mode is issued (Yes in Step S316), the magnetic field control instruction unit 45 performs the away magnetic field applying process of applying the uniform-gradient magnetic field in the away mode to the capsule endoscope 10 (Step S318).

Similarly to Steps S220 and S222 of FIG. 18, the behavior detector 243 performs the operation detection process (Step S320), and the gradient change instructing unit 248 determines whether the capsule endoscope 10 starts to leave the boundary surface based on the detection result of the behavior detector 243 (Step S322). When determining that the capsule endoscope 10 does not start to leave the boundary surface (No in Step S322), the gradient change instructing unit 248 issues the instruction to the magnetic field control instruction unit 45 to increase the gradient of the uniform-gradient magnetic field in the away mode by one step (Step S324) similarly to Step S224 of FIG. 18, and the flow goes to Step S318. The pieces of processing in Steps S318 to S324 are repeated until the gradient change instructing unit 248 determines that the capsule endoscope 10 starts to leave the boundary surface.

When determining that the capsule endoscope 10 starts to leave the boundary surface (Yes in Step S322), the gradient change instructing unit 248 performs the gradient update process of updating the gradient of the uniform-gradient magnetic field, which is generated in the last away magnetic field applying process by the magnetic field generating unit 2, as the gradient of the uniform-gradient magnetic field in the away mode (Step S326) similarly to Step S226 of FIG. 18.

Similarly to Steps S28 to S32 of FIG. 8, the magnetic field control instruction unit 45 performs the horizontal movement instruction determination process (Step S328), the horizontal movement position calculating process (Step S330), and the horizontal movement magnetic field applying process (Step S332). Similarly to Step S34 of FIG. 8, the magnetic field control instruction unit 45 performs the away mode stopping instruction determination process (Step S334). When determining that the instruction to stop the away mode is issued (Yes in Step S334), the magnetic field control instruction unit 45 performs the contact magnetic field applying process of applying the uniform-gradient magnetic field in the contact mode to the capsule endoscope 10 (Step S336), thereby returning the capsule endoscope 10 to the reference plane. On the other hand, when the magnetic field control instruction unit 45 determines that the instruction to stop the away mode is not issued (No in Step S334), the flow returns to Step S318 to continue the away mode.

Similarly to Step S38 of FIG. 8, the external control unit 304 determines whether the in-vivo observation is ended based on the instruction information input from an input unit 306 (Step S338). When determining that the in-vivo observation is not ended (No in Step S338), the external control unit 304 returns to Step S309 in order to continue the in-vivo observation. When the external control unit 304 determines that the in-vivo observation is ended (Yes in Step S338), the image receiving process of the image receiving unit 41 is ended (Step S340), and the image display process of the image display controller 42 is ended (Step S342). Then the external control unit 304 performs the image data storing process (Step S344), and the in-vivo observation is ended.

In the capsule medical device guidance system 301 according to the modification of the second embodiment, by performing the pieces of processing of FIG. 22, the position of the capsule endoscope 10 in the internal organ is detected, and the up mode or the down mode is automatically adjusted according to the position of the capsule endoscope 10, so that the burden of the gradient adjustment on the operator can be reduced.

The capsule endoscope 10 including the plural imaging units is used in the first and second embodiments. However, the monocular capsule endoscope including only the imaging unit 11A may be used.

The capsule endoscope 10 including the permanent magnet 19 is used in the first and second embodiments. However, the capsule endoscope including an electric magnet instead of the permanent magnet 19 may be used.

In the first and second embodiments, the magnetic field control instruction unit 45 causes the magnetic field generating unit 2 to generate the uniform-gradient magnetic field to control the magnetic attraction force that presses the capsule endoscope 10 against the boundary surface of the liquid or moves the capsule endoscope 10 away from the boundary surface. Alternatively, the peak magnetic field may be generated to control the magnetic attraction force that presses the capsule endoscope 10 against the boundary surface of the liquid or moves the capsule endoscope 10 away from the boundary surface.

The peak magnetic field generates the magnetic field having the horizontal peak to constrain the horizontal position of the capsule endoscope 10, and the peak magnetic field generates the vertical magnetic gradient near the peak to vertically guide the capsule endoscope 10. Accordingly, the magnetic field control instruction unit 45 controls the vertical magnetic gradient of the peak magnetic field generated by the magnetic field generating unit 2, thereby applying the magnetic field corresponding to the contact magnetic field applying process S14, S36, S214, S236, S314, and S336 and the away magnetic field applying process S18, S24, S54, S218, and S318 of FIGS. 8, 18, and 22 to the capsule endoscope 10.

The magnetic field control instruction unit 45 changes the horizontal position of the peak magnetic field generated by the magnetic field generating unit 2, thereby applying the magnetic field corresponding to the horizontal movement magnetic field applying process S32, S232, and S332 of FIGS. 8, 18, and 22 to the capsule endoscope 10.

Therefore, the horizontal position of the capsule endoscope 10 is constrained by the peak magnetic field, so that the guidance can more stably be performed in the liquid to improve the operability.

### EXPLANATIONS OF LETTERS OR NUMERALS

1, 201, 301 capsule medical device system
2 magnetic field generating unit
3 transmitting/receiving unit
4, 204, 304 external control unit
5 display unit
6, 206 input unit
7 storage unit
8 magnetic field controller
9 power supply unit
10 capsule endoscope
11A, 11B imaging unit
12 capsule-shaped casing
13A, 13B illumination unit
14A, 14B optical system
15A, 15B imaging element
16 wireless communication unit
16a antenna
17 control unit
18 power supply
19 permanent magnet
41 image receiving unit
42 image display controller
45 magnetic field control instruction unit
46, 346 magnetic field direction switching unit
47 magnetic field gradient storage unit
60, 260 operation input unit
243 behavior detector
248 gradient change instructing unit
344 position detector

## Claims

1. A capsule medical device guidance system (1; 201; 301) comprising:
a capsule medical device (10) that includes a magnetic field response unit;
a magnetic field generating unit (2) adapted to generate a magnetic field to the magnetic field response unit to guide the capsule medical device;
an operation input unit (60; 260) adapted to input operation information to magnetically guide the capsule medical device (10);
a control unit (8) adapted to control the magnetic field generating unit (2) to guide the capsule medical device (10) according to the operation information input from the operation input unit (60; 260); and
a selection unit (61) adapted to select one of a contact mode and an away mode, the capsule medical device (10) being brought into contact with a desired boundary surface in a plurality of boundary surfaces of a liquid in a subject in the contact mode, the capsule medical device (10) being moved away from the desired boundary surface in the away mode,
wherein the control unit (8) controls the magnetic field generating unit to continuously generate the magnetic field such that a total force in the liquid of a buoyant force of the capsule medical device (10), a gravity of the capsule medical device (10), and a magnetic attraction force is oriented toward the desired boundary surface side when the selection unit selects the contact mode, and the control unit (8) controls the magnetic field generating unit (2) such that the total force in the liquid of the buoyant force of the capsule medical device (10), the gravity of the capsule medical device (10), and the magnetic attraction force is oriented toward a direction other than the direction oriented toward the desired boundary surface side when the selection unit selects the away mode,
wherein the magnetic gradients of the magnetic fields which are generated when the contact and the away modes are selected are each a vertical magnetic gradient,
**characterized in that** the operation input unit (60; 260) includes a magnetic field direction switching instruction unit (46; 346) that issues an instruction to select either the boundary surface on a vertically upper side or the boundary surface on a vertically lower side as the boundary surface of the liquid which the capsule medical device (10) is brought into contact with or moved away from, and wherein control unit (8) is adapted to change the direction of the magnetic gradient of the magnetic field which is generated by the magnetic field generating unit (2) in each of the contact mode and the away mode according to the boundary surface that is selected by the magnetic field direction switching instruction unit (46; 346).

2. The capsule medical device guidance system according to claim 1, further comprising
a storage unit (7) that stores therein information on control of the magnetic field generating unit (2),
wherein the operation input unit (60; 260) further includes a gradient instruction unit that issues an instruction regarding an optimum condition of the magnetic gradient of the magnetic field which is generated when the away mode is selected,
the magnetic gradient of the magnetic field which is generated by the magnetic field generating unit (2) when the gradient instruction unit issues the instruction, is stored as the optimum condition in the storage unit, and
the control unit (8) causes the magnetic field generating unit (2) to generate the magnetic gradient of the optimum condition stored in the storage unit when the away mode is selected.

3. The capsule medical device guidance system according to claim 1, wherein
the magnetic field generating unit (2) generates a magnetic gradient in a different direction from the direction of the magnetic gradient which is generated when each of the contact mode and the away mode is selected,
the operation input unit (60; 260) further includes a magnetic field generating instruction unit that issues an instruction to generate the magnetic gradient in the different direction, and
the control unit (8) causes the magnetic field generating unit (2) to generate the magnetic gradient in the different direction while causing the magnetic field generating unit (2) to generate the magnetic gradient which is generated when the contact mode and the away mode are each selected, when the magnetic field generating instruction unit issues the instruction to generate the magnetic gradient in the different direction.

4. The capsule medical device guidance system according to claim 1, further comprising
a behavior detector (243) that detects a behavior of the capsule medical device (10),
wherein the control unit (8) changes the magnetic gradient which is generated when the away mode is selected, in a stepwise manner, sets the optimum magnetic gradient based on a detection result of the behavior detector, and causes the magnetic field generating unit (2) to generate the set optimum magnetic gradient.

5. The capsule medical device guidance system according to claim 1, wherein
a balance between the buoyant force of the capsule medical device (10) and the gravity of the capsule medical device (10) is substantially established, and
the control unit (8) causes the magnetic field generating unit (2) to generate a first magnetic field having a first magnetic gradient that brings the capsule medical device (10) into contact with the desired boundary surface when the selection unit selects the contact mode, and causes the magnetic field generating unit (2) to generate a second magnetic field having a second magnetic gradient that moves the capsule medical device (10) away from the desired boundary surface when the selection unit selects the away mode.

6. The capsule medical device guidance system according to claim 5, wherein
a direction of the first magnetic gradient is opposite to a direction of the second magnetic gradient.

7. The capsule medical device guidance system according to claim 1, wherein
a total force of the buoyant force of the capsule medical device (10) and the gravity of the capsule medical device (10) is oriented toward the desired boundary surface side, and
the control unit (8) causes the magnetic field generating unit (2) to generate a first magnetic field having a first magnetic gradient that moves the capsule medical device (10) away from the desired boundary surface when the selection unit selects the away mode, and causes the magnetic field generating unit (2) to stop the generation of the first magnetic field when the selection unit selects the contact mode.

8. The capsule medical device guidance system according to claim 7, wherein
a direction of the first magnetic gradient is opposite to the direction oriented toward the desired boundary surface side.

9. The capsule medical device guidance system according to claim 1, wherein
a total force of the buoyant force of the capsule medical device (10) and the gravity of the capsule medical device (10) is oriented toward a direction except the boundary surface side, and
the control unit (8) causes the magnetic field generating unit (2) to generate a first magnetic field having a first magnetic gradient that brings the capsule medical device (10) into contact with the desired boundary surface when the selection unit selects the contact mode, and causes the magnetic field generating unit (2) to stop the generation of at least the first magnetic field when the selection unit selects the away mode.

10. The capsule medical device guidance system according to claim 9, wherein
a direction of the first magnetic gradient is opposite to the direction oriented toward the desired boundary surface side.

11. The capsule medical device guidance system according to claim 7, wherein
a direction of the first magnetic gradient is a vertical magnetic gradient.

12. The capsule medical device guidance system according to claim 9, wherein
a direction of the first magnetic gradient is a vertical magnetic gradient.

## Patentansprüche

1. System zum Führen einer verkapselten medizinischen Vorrichtung (1; 201; 301), umfassend:
eine medizinische Kapselvorrichtung (10), die eine Magnetfeldreaktionseinheit umfasst;
eine Magnetfelderzeugungseinheit (2), die dafür ausgebildet ist, ein Magnetfeld für die Magnetfeldreaktionseinheit zu erzeugen, um die medizinische Kapselvorrichtung zu führen;
eine Bedienungseingabeeinheit (60; 260), die dafür ausgebildet ist, Bedienungsinformation einzugeben, um die medizinische Kapselvorrichtung (10) magnetisch zu führen;
eine Steuereinheit (8), die dafür ausgebildet ist, die Magnetfelderzeugungseinheit (2) derart zu steuern, dass sie die medizinische Kapselvorrichtung (10) entsprechend der von der Bedienungseingabeeinheit (60; 260) eingegebenen Bedienungsinformation führt; und
eine Auswahleinheit (61), die dafür ausgebildet ist, einen eines Kontaktmodus und eines Entferntmodus auszuwählen, wobei die medizinische Kapselvorrichtung (10) in dem Kontaktmodus mit einer erwünschten Grenzfläche in einer Mehrzahl von Grenzflächen einer Flüssigkeit in einem Patienten in Kontakt gebracht wird, wobei die medizinische Kapselvorrichtung (10) in dem Entferntmodus von der erwünschten Grenzfläche wegbewegt wird,
wobei die Steuereinheit (8) die Magnetfelderzeugungseinheit derart steuert, dass das Magnetfeld kontinuierlich derart erzeugt wird, dass eine Gesamtkraft in der Flüssigkeit einer Auftriebskraft der medizinischen Kapselvorrichtung (10), eine Schwerkraft der medizinischen Kapselvorrichtung (10) und eine magnetische Anziehungskraft in Richtung der erwünschten Grenzflächenseite ausgerichtet sind, wenn die Auswahleinheit den Kontaktmodus auswählt, und die Steuereinheit (8) die Magnetfelderzeugungseinheit (2) derart steuert, dass die Gesamtkraft in der Flüssigkeit der Auftriebskraft der medizinischen Kapselvorrichtung (10), die Schwerkraft der medizinischen Kapselvorrichtung (10) und die magnetische Anziehungskraft in eine andere Richtung als die Richtung, die in Richtung der erwünschten Grenzflächenseite ausgerichtet ist, ausgerichtet sind, wenn die Auswahleinheit den Entferntmodus auswählt,
wobei die Magnetgradienten der Magnetfelder, die erzeugt werden, wenn der Kontaktmodus und der Entferntmodus ausgewählt werden, jeweils ein vertikaler Magnetgradient sind,
**dadurch gekennzeichnet, dass** die Bedienungseingabeeinheit (60; 260) eine Magnetfeldrichtungsumschaltbefehlseinheit (46; 346) umfasst, die einen Befehl zur Auswahl entweder der Grenzfläche auf einer vertikalen Oberseite oder der Grenzfläche auf einer vertikalen Unterseite als die Grenzfläche der Flüssigkeit ausgibt, mit der die medizinische Kapselvorrichtung (10) in Kontakt gebracht wird oder von der die medizinische Kapselvorrichtung (10) wegbewegt wird, und
wobei die Steuereinheit (8) dafür ausgebildet ist, die Richtung des Magnetgradienten des Magnetfelds zu ändern, das von der Magnetfelderzeugungseinheit (2) in jedem des Kontaktmodus und des Entferntmodus gemäß der Grenzfläche, die von der Magnetfeldrichtungsumschaltbefehlseinheit (46; 346) ausgewählt ist, erzeugt wird.

2. System zum Führen einer verkapselten medizinischen Vorrichtung nach Anspruch 1, das ferner umfasst
eine Speichereinheit (7), die darin Information über die Steuerung der Magnetfelderzeugungseinheit (2) speichert,
wobei die Bedienungseingabeeinheit (60; 260) ferner eine Gradientenbefehlseinheit umfasst, die einen Befehl bezüglich einer optimalen Bedingung des Magnetgradienten des Magnetfelds ausgibt, das erzeugt wird, wenn der Entferntmodus ausgewählt ist,
der Magnetgradient des Magnetfelds, das von der Magnetfelderzeugungseinheit (2) erzeugt wird, wenn die Gradientenbefehlseinheit den Befehl ausgibt, als die optimale Bedingung in der Auswahleinheit gespeichert wird und
die Steuereinheit (8) bewirkt, dass die Magnetfelderzeugungseinheit (2) den Magnetgradienten der optimalen Bedingung, die in der Auswahleinheit gespeichert wird, wenn der Entferntmodus ausgewählt ist, erzeugt.

3. System zum Führen einer verkapselten medizinischen Vorrichtung nach Anspruch 1, wobei
die Magnetfelderzeugungseinheit (2) einen Magnetgradienten in eine andere Richtung erzeugt als die Richtung des Magnetgradienten, der erzeugt wird, wenn jeder des Kontaktmodus und des Entferntmodus ausgewählt ist,
die Bedienungseingabeeinheit (60; 260) ferner eine Magnetfelderzeugungsbefehlseinheit umfasst, die einen Befehl ausgibt, den Magnetgradienten in die andere Richtung zu erzeugen, und
die Steuereinheit (8) bewirkt, dass die Magnetfelderzeugungseinheit (2) den Magnetgradienten in die andere Richtung erzeugt, während sie bewirkt, dass die Magnetfelderzeugungseinheit (2) den Magnetgradienten erzeugt, der erzeugt wird, wenn jeweils der Kontaktmodus und der Entferntmodus ausgewählt sind, wenn die Magnetfelderzeugungsbefehlseinheit den Befehl ausgibt, den Magnetgradienten in die andere Richtung zu erzeugen.

4. System zum Führen einer verkapselten medizinischen Vorrichtung nach Anspruch 1, das ferner umfasst
einen Verhaltensdetektor (243), der ein Verhalten der medizinischen Kapselvorrichtung (10) erfasst,
wobei die Steuereinheit (8) den Magnetgradienten, der erzeugt wird, wenn der Entferntmodus ausgewählt ist, stufenweise ändert, den optimalen Magnetgradienten auf Grundlage eines Erfassungsergebnisses des Verhaltensdetektors einstellt und bewirkt, dass die Magnetfelderzeugungseinheit (2) den eingestellten optimalen Magnetgradienten erzeugt.

5. System zum Führen einer verkapselten medizinischen Vorrichtung nach Anspruch 1, wobei
ein Gleichgewicht zwischen der Auftriebskraft der medizinischen Kapselvorrichtung (10) und der Schwerkraft der medizinischen Kapselvorrichtung (10) im Wesentlichen geschaffen wird, und
die Steuereinheit (8) bewirkt, dass die Magnetfelderzeugungseinheit (2) ein erstes Magnetfeld mit einem ersten Magnetgradienten erzeugt, das die medizinische Kapselvorrichtung (10) mit der erwünschten Grenzfläche in Kontakt bringt, wenn die Auswahleinheit den Kontaktmodus auswählt, und bewirkt, dass die Magnetfelderzeugungseinheit (2) ein zweites Magnetfeld mit einem zweiten Magnetgradienten erzeugt, das die medizinische Kapselvorrichtung (10) von der erwünschten Grenzfläche wegbewegt, wenn die Auswahleinheit den Entferntmodus auswählt.

6. System zum Führen einer verkapselten medizinischen Vorrichtung nach Anspruch 5, wobei
eine Richtung des ersten Magnetgradienten einer Richtung des zweiten Magnetgradienten entgegengesetzt ist.

7. System zum Führen einer verkapselten medizinischen Vorrichtung nach Anspruch 1, wobei
eine Gesamtkraft der Auftriebskraft der medizinischen Kapselvorrichtung (10) und der Schwerkraft der medizinischen Kapselvorrichtung (10) in Richtung der erwünschten Grenzflächenseite ausgerichtet ist, und
die Steuereinheit (8) bewirkt, dass die Magnetfelderzeugungseinheit (2) ein erstes Magnetfeld mit einem ersten Magnetgradienten erzeugt, das die medizinische Kapselvorrichtung (10) von der erwünschten Grenzfläche wegbewegt, wenn die Auswahleinheit den Entferntmodus auswählt, und bewirkt, dass die Magnetfelderzeugungseinheit (2) die Erzeugung des ersten Magnetfelds beendet, wenn die Auswahleinheit den Kontaktmodus auswählt.

8. System zum Führen einer verkapselten medizinischen Vorrichtung nach Anspruch 7, wobei
eine Richtung des ersten Magnetgradienten der Richtung, die in Richtung der erwünschten Grenzflächenseite ausgerichtet ist, entgegengesetzt ist.

9. System zum Führen einer verkapselten medizinischen Vorrichtung nach Anspruch 1, wobei
eine Gesamtkraft der Auftriebskraft der medizinischen Kapselvorrichtung (10) und der Schwerkraft der medizinischen Kapselvorrichtung (10) in eine Richtung außer der Richtung der Grenzflächenseite ausgerichtet ist, und
die Steuereinheit (8) bewirkt, dass die Magnetfelderzeugungseinheit (2) ein erstes Magnetfeld mit einem ersten Magnetgradienten erzeugt, das die medizinische Kapselvorrichtung (10) mit der erwünschten Grenzfläche in Kontakt bringt, wenn die Auswahleinheit den Kontaktmodus auswählt, und bewirkt, dass die Magnetfelderzeugungseinheit (2) die Erzeugung mindestens des ersten Magnetfelds beendet, wenn die Auswahleinheit den Entferntmodus auswählt.

10. System zum Führen einer verkapselten medizinischen Vorrichtung nach Anspruch 9, wobei
eine Richtung des ersten Magnetgradienten der Richtung, die in Richtung der erwünschten Grenzflächenseite ausgerichtet ist, entgegengesetzt ist.

11. System zum Führen einer verkapselten medizinischen Vorrichtung nach Anspruch 7, wobei
eine Richtung des ersten Magnetgradienten ein vertikaler Magnetgradient ist.

12. System zum Führen einer verkapselten medizinischen Vorrichtung nach Anspruch 9, wobei
eine Richtung des ersten Magnetgradienten ein vertikaler Magnetgradient ist.

## Revendications

1. Système de guidage de dispositif médical à capsule (1 ; 201 ; 301) comprenant :
un dispositif médical à capsule (10) qui comprend une unité de réponse de champ magnétique ;
une unité de génération de champ magnétique (2) apte à générer un champ magnétique pour l'unité de réponse de champ magnétique afin de guider le dispositif médical à capsule ;
une unité d'entrée de fonctionnement (60 ; 260) apte à entrer des informations de fonctionnement afin de guider magnétiquement le dispositif médical à capsule (10) ;
une unité de commande (8) apte à commander l'unité de génération de champ magnétique (2) afin de guider le dispositif médical à capsule (10) conformément aux informations de fonctionnement entrées à partir de l'unité d'entrée de fonctionnement (60 ; 260) ; et
une unité de sélection (61) apte à sélectionner l'un d'un mode de contact et d'un mode à distance, le dispositif médical à capsule (10) étant mis en contact avec une surface limite souhaitée parmi une pluralité de surfaces limites d'un liquide dans un sujet dans le mode de contact, le dispositif médical à capsule (10) étant déplacé à distance de la surface limite souhaitée dans le mode à distance,
l'unité de commande (8) commandant l'unité de génération de champ magnétique pour générer de manière continue le champ magnétique de telle sorte qu'une force totale dans le liquide d'une poussée d'Archimède du dispositif médical à capsule (10), d'une pesanteur du dispositif médical à capsule (10) et d'une force d'attraction magnétique est orientée vers le côté surface limite souhaitée lorsque l'unité de sélection sélectionne le mode de contact, et l'unité de commande (8) commandant l'unité de génération de champ magnétique (2) de telle sorte que la force totale dans le liquide de la poussée d'Archimède du dispositif médical à capsule (10), de la pesanteur du dispositif médical à capsule (10) et de la force d'attraction magnétique est orientée dans une direction autre que la direction orientée vers le côté surface limite souhaitée lorsque l'unité de sélection sélectionne le mode à distance,
les gradients magnétiques des champs magnétiques qui sont générés lorsque les modes de contact et à distance sont sélectionnés étant chacun un gradient magnétique vertical,
**caractérisé par le fait que** l'unité d'entrée de fonctionnement (60 ; 260) comprend une unité d'instruction de commutation de direction de champ magnétique (46 ; 346) qui émet une instruction pour sélectionner soit la surface limite sur un côté verticalement supérieur, soit la surface limite sur un côté verticalement inférieur, en tant que surface limite du liquide avec laquelle le dispositif médical à capsule (10) est mis en contact ou à distance de laquelle il est déplacé, et
l'unité de commande (8) est apte à changer la direction du gradient magnétique du champ magnétique qui est généré par l'unité de génération de champ magnétique (2) dans chacun du mode de contact et du mode à distance conformément à la surface limite qui est sélectionnée par l'unité d'instruction de commutation de direction de champ magnétique (46 ; 346).

2. Système de guidage de dispositif médical à capsule selon la revendication 1, comprenant en outre :
une unité de stockage (7) qui stocke à l'intérieur de celle-ci des informations sur la commande de l'unité de génération de champ magnétique (2),
l'unité d'entrée de fonctionnement (60 ; 260) comprenant en outre une unité d'instruction de gradient qui émet une instruction concernant un état optimal du gradient magnétique du champ magnétique qui est généré lorsque le mode à distance est sélectionné,
le gradient magnétique du champ magnétique qui est généré par l'unité de génération de champ magnétique (2) lorsque l'unité d'instruction de gradient émet l'instruction, étant stocké en tant qu'état optimal dans l'unité de stockage, et
l'unité de commande (8) amenant l'unité de génération de champ magnétique (2) à générer le gradient magnétique de l'état optimal stocké dans l'unité de stockage lorsque le mode à distance est sélectionné.

3. Système de guidage de dispositif médical à capsule selon la revendication 1, dans lequel :
l'unité de génération de champ magnétique (2) génère un gradient magnétique dans une direction différente de la direction du gradient magnétique qui est généré lorsque chacun du mode de contact et du mode à distance est sélectionné,
l'unité d'entrée de fonctionnement (60 ; 260) comprend en outre une unité d'instruction de génération de champ magnétique qui émet une instruction pour générer le gradient magnétique dans la direction différente, et
l'unité de commande (8) amène l'unité de génération de champ magnétique (2) à générer le gradient magnétique dans la direction différente tout en amenant l'unité de génération de champ magnétique (2) à générer le gradient magnétique qui est généré lorsque le mode de contact et le mode à distance sont chacun sélectionnés, lorsque l'unité d'instruction de génération de champ magnétique émet l'instruction de générer le gradient magnétique dans la direction différente.

4. Système de guidage de dispositif médical à capsule selon la revendication 1, comprenant en outre :
un détecteur de comportement (243) qui détecte un comportement du dispositif médical à capsule (10),
l'unité de commande (8) changeant le gradient magnétique qui est généré lorsque le mode à distance est sélectionné, d'une manière progressive, réglant le gradient magnétique optimal sur la base d'un résultat de détection du détecteur de comportement, et amenant l'unité de génération de champ magnétique (2) à générer le gradient magnétique optimal réglé.

5. Système de guidage de dispositif médical à capsule selon la revendication 1, dans lequel :
un équilibre entre la poussée d'Archimède du dispositif médical à capsule (10) et la pesanteur du dispositif médical à capsule (10) est établi de manière substantielle, et
l'unité de commande (8) amène l'unité de génération de champ magnétique (2) à générer un premier champ magnétique ayant un premier gradient magnétique qui met le dispositif médical à capsule (10) en contact avec la surface limite souhaitée lorsque l'unité de sélection sélectionne le mode de contact, et amène l'unité de génération de champ magnétique (2) à générer un second champ magnétique ayant un second gradient magnétique qui déplace le dispositif médical à capsule (10) à distance de la surface limite souhaitée lorsque l'unité de sélection sélectionne le mode à distance.

6. Système de guidage de dispositif médical à capsule selon la revendication 5, dans lequel :
une direction du premier gradient magnétique est opposée à une direction du second gradient magnétique.

7. Système de guidage de dispositif médical à capsule selon la revendication 1, dans lequel :
une force totale de la poussée d'Archimède du dispositif médical à capsule (10) et de la pesanteur du dispositif médical à capsule (10) est orientée vers le côté surface limite souhaitée, et
l'unité de commande (8) amène l'unité de génération de champ magnétique (2) à générer un premier champ magnétique ayant un premier gradient magnétique qui déplace le dispositif médical à capsule (10) à distance de la surface limite souhaitée lorsque l'unité de sélection sélectionne le mode à distance, et amène l'unité de génération de champ magnétique (2) à arrêter la génération du premier champ magnétique lorsque l'unité de sélection sélectionne le mode de contact.

8. Système de guidage de dispositif médical à capsule selon la revendication 7, dans lequel :
une direction du premier gradient magnétique est opposée à la direction orientée vers le côté surface limite souhaitée.

9. Système de guidage de dispositif médical à capsule selon la revendication 1, dans lequel :
une force totale de la poussée d'Archimède du dispositif médical à capsule (10) et de la pesanteur du dispositif médical à capsule (10) est orientée vers une direction à l'exception du côté surface limite, et
l'unité de commande (8) amène l'unité de génération de champ magnétique (2) à générer un premier champ magnétique ayant un premier gradient magnétique qui met le dispositif médical à capsule (10) en contact avec la surface limite souhaitée lorsque l'unité de sélection sélectionne le mode de contact, et amène l'unité de génération de champ magnétique (2) à arrêter la génération d'au moins le premier champ magnétique lorsque l'unité de sélection sélectionne le mode à distance.

10. Système de guidage de dispositif médical à capsule selon la revendication 9, dans lequel :
une direction du premier gradient magnétique est opposée à la direction orientée vers le côté surface limite souhaitée.

11. Système de guidage de dispositif médical à capsule selon la revendication 7, dans lequel :
une direction du premier gradient magnétique est un gradient magnétique vertical.

12. Système de guidage de dispositif médical à capsule selon la revendication 9, dans lequel :
une direction du premier gradient magnétique est un gradient magnétique vertical.
